# EUROPEAN PATENT SPECIFICATION

(11) **EP 3 142 656 B1**
(45) Date of publication and mention of the grant of the patent: **17.10.2018**
(21) Application number: 16740695.8
(22) Date of filing: 20.01.2016
(51) Int. Cl.: A61K 9/127, A61K 9/00, A61K 31/195, A61P 7/04

(54) **MULTIVESICULAR LIPOSOME FORMULATIONS OF TRANEXAMIC ACID**
MULTIVESIKULÄRE LIPOSOMFORMULIERUNGEN VON TRANEXAMSÄURE
FORMULATIONS DE LIPOSOMES MULTIVÉSICULAIRES D'ACIDE TRANEXAMIQUE

(30) Priority: 21.01.2015 US 201562106067 P
(43) Date of publication of application: 22.03.2017
(73) Proprietor: Pacira Pharmaceuticals, Inc., San Diego, California 92121 (US)
(72) Inventor: LOS, Kathleen Dunne Albright, San Diego, CA 92117 (US); GARCIA, Louie Daniel, San Diego, CA 92128 (US); KURZ, Stephanie, San Diego, CA 92102 (US); KHARITONOV, Vladimir, San Diego, CA 92122 (US)
(74) Representative: Bassil, Nicholas Charles
(86) International application number: PCT/US2016/014169
(87) International publication number: WO 2016/118652

(56) References cited:
- US-A- 5 766 627
- US-A- 5 997 899
- US-A- 6 132 766
- US-A1- 2003 068 365
- US-A1- 2012 010 557
- US-A1- 2012 010 557
- MANOSROI A ET AL: "Stability and release of topical tranexamic acid liposome formulations", JOURNAL OF COSMETIC SCIENCE, vol. 53, no. 6, November 2002 (2002-11), pages 375-386, XP055349080, US ISSN: 1525-7886

## Description

### BACKGROUND

### Field

The present application relates to multivesicular liposome (MVL) formulations of tranexamic acid (TXA) which minimize the side effects of TXA while maintaining or improving efficacy and prolonging the therapeutic effect of the TXA. In particular, embodiments of the present application relate to compositions comprising TXA encapsulated multivesicular liposomes, processes of making the same, and methods of administration of the same. Methods of making multivesicular liposomes containing TXA and their use as medicaments are also provided.

Tranexamic acid (TXA) is a synthetic analog of the amino acid lysine that is used to treat or prevent excessive blood loss after trauma, during surgery, or during menstruation. TXA is an anti-fibrinolytic that exerts its clotting effect through the reversible blockade of lysine binding sites on plasminogen molecules. Intravenously administered tranexamic acid caused reductions relative to placebo of 29 to 54% in postoperative blood losses in patients undergoing cardiac surgery with cardiopulmonary bypass, with statistically significant reductions in transfusion requirements in some studies. Meta-analysis of 60 trials showed TXA and aprotinin, unlike epsilon-aminocaproic acid and desmopressin, reduced significantly the number of patients requiring allogeneic blood transfusions after cardiac surgery with CPB. TXA also significantly reduced mean blood losses after oral surgery in patients with hemophilia. Reductions in blood loss were also obtained with the use of the drug in patients undergoing orthotopic liver transplantation or transurethral prostatic surgery. Clinical benefit has also been reported with TXA in patients with hereditary angioneurotic edema. *See, e.g.,* Dunn and Goa, Drugs, Vol. 57, No. 6, pages 1005-32 (1999).

However, oral administration of TXA often results in gastrointestinal discomfort, and intravenous administration can cause dizziness and hypotension. These side-effects are primarily due to the rapid increase in plasma levels of TXA when administered as a bolus dose sufficient to provide the necessary clotting effect. However, sustained intravenous administration is not always feasible, particularly for trauma patients. Moreover, the rapid increases in TXA plasma levels are followed by a rapid decrease in TXA concentration due to excretion and metabolism. This rapid decrease quickly reaches a level at which TXA is present in sub-therapeutic amounts. Accordingly, there is a need for a stable, sustained release formulation of TXA for both topical and subcutaneous application. In addition, there is a need for a stable formulation comprising both sustained release and immediate release TXA for both topical and various parenteral applications, such as subcutaneous injection, wound infiltration or wound instillation.

### BRIEF DESCRIPTION OF THE FIGURES

Fig. 1A illustrates the decrease in plasma levels of free TXA in saline solution, TXA-MVL, and Depo-TXA over 96 hours as described in the animal study of Example 1.
Fig. 1B illustrates the percent of total area under the curve (AUC) of free TXA in saline solution, TXA-MVL, and Depo-TXA for up to 96 hours after administration as described in the animal study of Example 1.
Figs. 2-4, 5A-5B, 6A-6B, 7A-7B, and 8A-8B refer to pharmacokinetic data obtained from animal studies, described in Example 3.

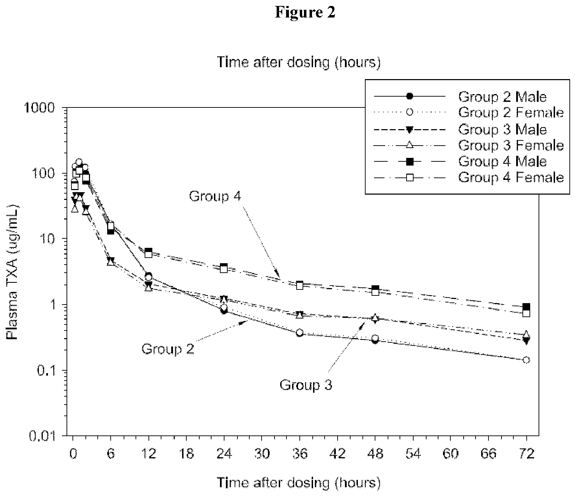

Fig. 2 illustrates mean plasma concentrations of TXA over 72 hours post-injection on treatment day 1. Group 2 was administered 120 mg/kg TXA, Group 3 was administered 40 mg/kg Depo-TXA, and Group 4 was administered 120 mg/kg Depo-TXA.

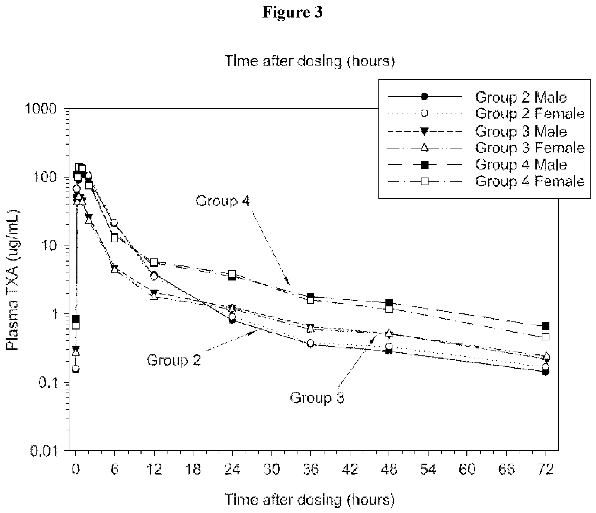

Fig. 3 illustrates mean plasma concentrations of TXA over 72 hours after administration on treatment day 10. Group 2 was administered 120 mg/kg TXA, Group 3 was administered 40 mg/kg Depo-TXA, and Group 4 was administered 120 mg/kg Depo-TXA.

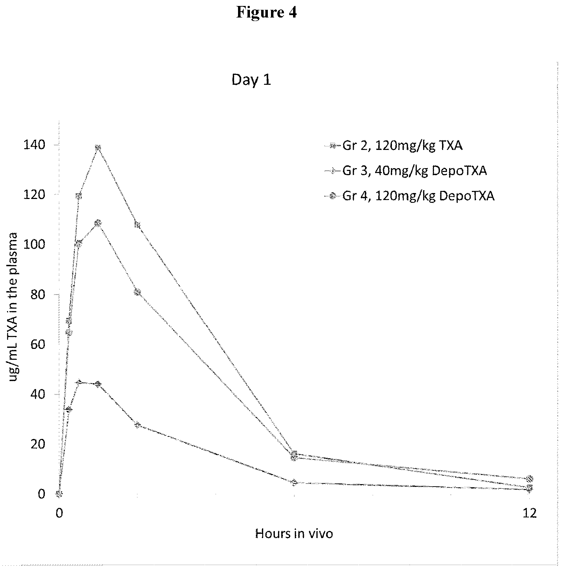

Fig. 4 illustrates the concentration of TXA in plasma over 12 hours after administration on treatment day 1 for Groups 2, 3 and 4.

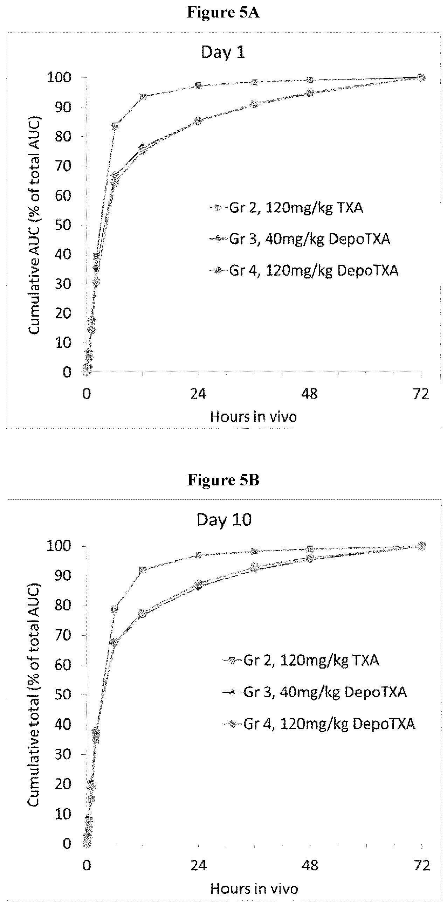

Fig. 5A illustrates the percent of total area under the curve (AUC) of TXA for up to 72 hours after administration on treatment day 1 for Groups 2, 3 and 4.
Fig. 5B illustrates the percent of total area under the curve (AUC) of TXA for up to 72 hours after administration on treatments day 10 for Groups 2, 3 and 4.

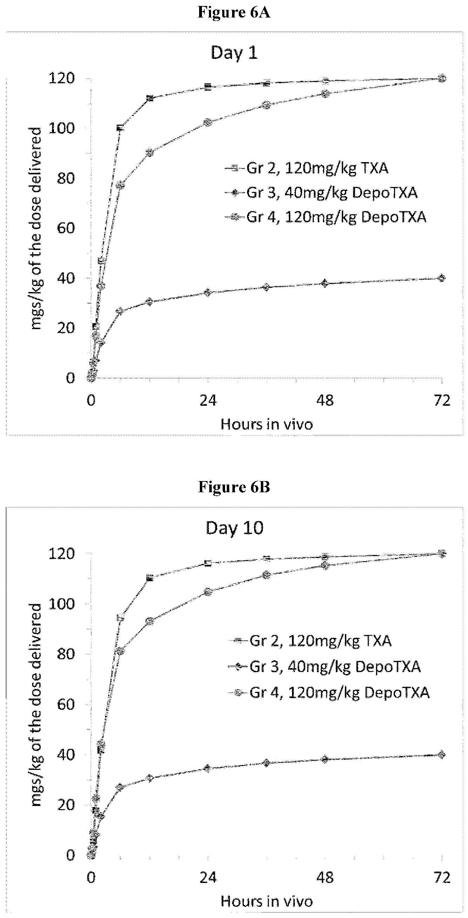

Fig. 6A illustrates the total amount of TXA delivered over 72 hours after administration on treatment day 1 for Groups 2, 3 and 4.
Fig. 6B illustrates the total amount of TXA delivered over 72 hours after administration on treatment day 10 for Groups 2, 3 and 4.

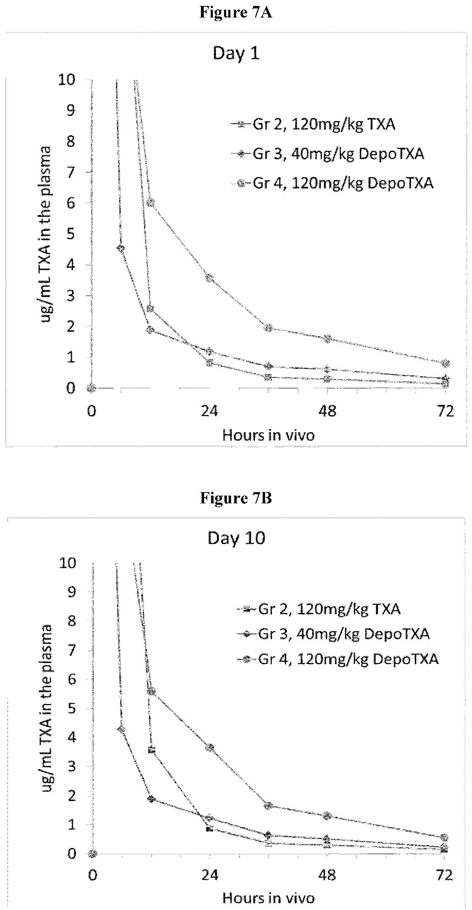

Fig. 7A illustrates the decrease in plasma levels of TXA over 72 hours after administration on day 1 for Groups 2, 3 and 4.
Fig. 7B illustrates the decrease in plasma levels of TXA over 72 hours after administration on day 10 for Groups 2, 3 and 4.

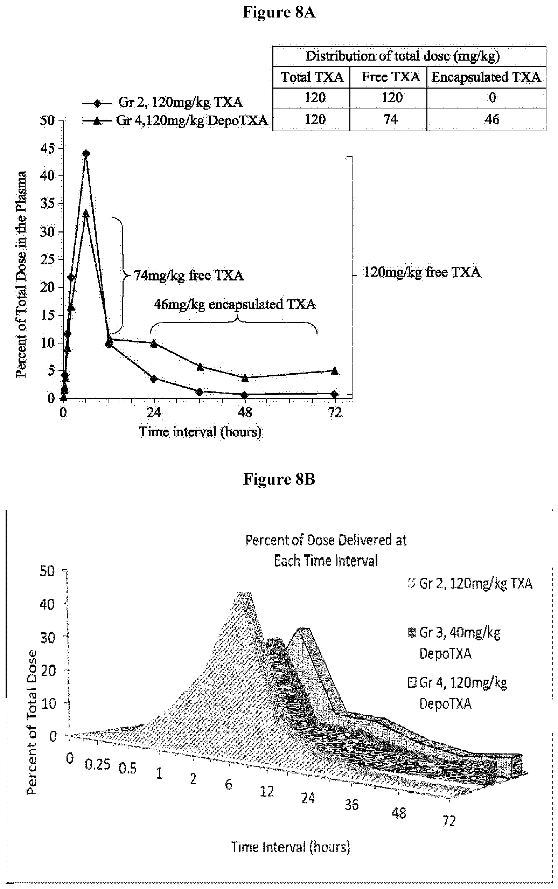

Fig. 8A illustrates the percent of the total TXA dose in plasma over 72 hours after administration for Groups 2 and 4.
Fig. 8B illustrates the percentage of the TXA dose in the plasma over 72 hours after administration for Groups 2, 3, and 4.

### SUMMARY

The present invention is directed to a pharmaceutical composition comprising multivesicular liposomes encapsulating tranexamic acid, said multivesicular liposomes comprising: tranexamic acid; a lipid component comprising at least one amphipathic lipid and at least one neutral lipid; and one or more pH modifying agents.

The present invention is also directed to the aforementioned pharmaceutical composition for use in treating, ameliorating or preventing blood loss. Some embodiments of the present application are related to pharmaceutical compositions comprising: multivesicular liposomes encapsulating tranexamic acid comprising tranexamic acid, a lipid component comprising at least one amphipathic lipid and at least one neutral lipid, and one or more pH modifying agents; and unencapsulated tranexamic acid. In some embodiments, the concentration of total tranexamic acid in the pharmaceutical composition is from about 1 mg/mL to about 80 mg/mL and the unencapsulated tranexamic acid is about 1% to about 80% of the total amount of tranexamic acid in the pharmaceutical composition.

Some embodiments of the present application are related to methods of treating, ameliorating or preventing blood loss comprising administering a pharmaceutical composition comprising multivesicular liposomes encapsulating tranexamic acid, the multivesicular liposomes comprising tranexamic acid, a lipid component comprising at least one amphipathic lipid and at least one neutral lipid, and one or more pH modifying agents; and unencapsulated tranexamic acid.

Some other embodiments of the present application are related to processes for preparing multivesicular liposomes comprising tranexamic acid, said process comprising: preparing a first aqueous component comprising tranexamic acid and at least one pH modifying agent; preparing a lipid component comprising at least one organic solvent, at least one amphipathic lipid, and at least one neutral lipid; mixing said first aqueous component and said lipid component to form a water-in-oil emulsion, wherein at least one component comprises tranexamic acid; contacting said water-in-oil emulsion with a second aqueous component to form solvent-containing spherules; and removing the organic solvent from the solvent-containing spherules to form multivesicular liposomes. In some embodiments, the process further comprises an additional step of suspending the multivesicular liposome in a solution comprising free tranexamic acid to form a pharmaceutical composition comprising both encapsulated and unencapsulated tranexamic acid.

Some other embodiments of the present application are related pharmaceutical compositions comprising tranexamic acid containing multivesicular liposomes prepared by the process described herein.

Some embodiments provide a pharmaceutical composition comprising multivesicular liposomes encapsulating tranexamic acid, said multivesicular liposomes comprising: tranexamic acid; a lipid component comprising at least one amphipathic lipid and at least one neutral lipid; and one or more pH modifying agents; and unencapsulated tranexamic acid. Some embodiments provide a pharmaceutical composition comprising multivesicular liposomes encapsulating tranexamic acid, said multivesicular liposomes comprising: tranexamic acid; a lipid component comprising at least one amphipathic lipid and at least one neutral lipid; and one or more pH modifying agents.

In some embodiments, the multivesicular liposomes further comprise one or more osmotic agents and/or density modifying agents. In some embodiments, the multivesicular liposomes further comprise cholesterol and/or a plant sterol. In some embodiments, the amphipathic lipid comprises phosphatidylcholine, or phosphatidylglycerol or salts thereof, or combinations thereof. In some embodiments, the phosphatidylglycerol is DPPG. In some embodiments, the phosphatidylcholine is selected from DEPC or DOPC, or a combination thereof.

In some embodiments, the neutral lipid comprises triglyceride, propylene glycol ester, ethylene glycol ester, or squalene, or combinations thereof. In some embodiments, the neutral lipid comprises triglyceride. In some embodiments, the triglyceride is selected from triolein or tricaprylin, or a combination thereof.

In some embodiments, said pH modifying agents are selected from organic acids, organic bases, inorganic acids, or inorganic bases, or combinations thereof. In some embodiments, said pH modifying agents are selected from inorganic acids, or organic bases, or combinations thereof. In some embodiments, said pH modifying agents are selected from organic acids, or organic bases, or combinations thereof. In some embodiments, the inorganic acid is selected from hydrochloric acid or phosphoric acid. In some embodiments, the organic acid is selected from tartaric acid, or glutamic acid, or a combination thereof. In some embodiments, the organic base is selected from histidine, arginine, lysine, or tromethamine, or combinations thereof.

In some embodiments, the concentration of total tranexamic acid in the pharmaceutical composition is from about 1 mg/mL to about 80 mg/mL. In some embodiments, the concentration of total tranexamic acid in the pharmaceutical composition is from about 2.5 mg/mL to about 40 mg/mL. In some embodiments, the concentration of total tranexamic acid in the pharmaceutical composition is from about 5 mg/mL to about 25 mg/mL. In some embodiments, the concentration of total tranexamic acid in the pharmaceutical composition is from about 10 mg/mL to about 20 mg/mL.

In some embodiments, the unencapsulated tranexamic acid is about 1% to about 80% of the total amount of tranexamic acid in the pharmaceutical composition. In some embodiments, the unencapsulated tranexamic acid is about 20% to about 70% of the total amount of tranexamic acid in the pharmaceutical composition. In some embodiments, the unencapsulated tranexamic acid is about 30% to about 60% of the total amount of tranexamic acid in the pharmaceutical composition.

In some embodiments, the unencapsulated tranexamic acid is about 50% of the total amount of tranexamic acid in the pharmaceutical composition. In some embodiments, the unencapsulated tranexamic acid is less than about 10% of the total tranexamic acid in the pharmaceutical composition.

In some embodiments, said multivesicular liposomes have an external pH range from about 4.0 to about 9.0. In some embodiments, said external pH range is from about 4.5 to about 8.5. In some embodiments, said multivesicular liposomes have an internal pH range of about 3.0 to about 9.0. In some embodiments, said internal pH range is from about 3.5 to about 5.5.

In some embodiments, the tranexamic acid encapsulated multivesicular liposomes are stable at 37 °C for at least 2 days.

Some embodiments provide methods for treating, ameliorating or preventing blood loss comprising administering a pharmaceutical composition, as described herein, to a subject in need thereof.

In some embodiments, the administration is parenteral. In some embodiments, the parenteral administration is selected from subcutaneous injection, tissue injection, wound infiltration, or wound instillation. In some embodiments, the parenteral administration is subcutaneous injection. In some embodiments, the parenteral administration is tissue injection. In some embodiments, the parenteral administration is wound infiltration. In some embodiments, the parenteral administration is wound installation.

In some embodiments, the administration is topical. In some embodiments, the administration is both topical and parenteral. In some embodiments, the topical administration comprises direct contacting said pharmaceutical composition with a cavity or a surface of the subject body that is in need of treatment.

Some embodiments provide a process for preparing multivesicular liposomes comprising tranexamic acid, comprising preparing a first aqueous component comprising tranexamic acid and at least one pH modifying agent; preparing a lipid component comprising at least one organic solvent, at least one amphipathic lipid, and at least one neutral lipid; mixing said first aqueous component and said lipid component to form a water-in-oil emulsion, wherein at least one component comprises tranexamic acid; contacting said water-in-oil emulsion with a second aqueous component to form solvent-containing spherules; and removing the organic solvent from the solvent-containing spherules to form multivesicular liposomes. Some embodiments further comprise suspending the multivesicular liposomes in a solution comprising saline to form a pharmaceutical composition comprising encapsulated tranexamic acid.

Some embodiments further comprise suspending the multivesicular liposomes in a solution comprising tranexamic acid to form a pharmaceutical composition comprising both encapsulated and unencapsulated tranexamic acid.

In some embodiments, the lipid component further comprises cholesterol and/or a plant sterol. In some embodiments, the amphipathic lipid comprises phosphatidylcholine, or phosphatidylglycerol or salts thereof, or combinations thereof. In some embodiments, the phosphatidylglycerol is DPPG. In some embodiments, the phosphatidylcholine is selected from DEPC or DOPC, or a combination thereof. In some embodiments, the neutral lipid comprises triglyceride, propylene glycol ester, ethylene glycol ester, or squalene, or combinations thereof. In some embodiments, the neutral lipid comprises triglyceride. In some embodiments, the triglyceride is selected from triolein or tricaprylin, or a combination thereof.

In some embodiments, the first aqueous component further comprises at least one osmotic agent and/or a density modifying agent. In some embodiments, the pH modifying agent of the first aqueous component is selected from an inorganic acid, an organic acid, an inorganic base, or an organic base, or combinations thereof.

In some embodiments, said pH modifying agent is selected from hydrochloric acid, phosphoric acid, or tartaric acid, or combinations thereof. In some embodiments, said pH modifying agent is selected from histidine, arginine, tromethamine, or combinations thereof.

In some embodiments, the pH range of the first aqueous component is from about 2.0 to about 9.0. In some embodiments, the pH range of the first aqueous component is from about 3.5 to about 5.5. In some embodiments, the pH range of the first aqueous component is from about 4.3 to about 5.5. In some embodiments, the pH range of the first aqueous component is from about 7.5 to about 9.0. In some embodiments, the pH of the first aqueous component is about 7.7.

In some embodiments, said second aqueous component comprises at least one osmotic agent, and at least one pH modifying agent.

In some embodiments, the pH range of the second aqueous component is from about 3.5 to about 10.5. In some embodiments, the pH range of the second aqueous component is from about 7.5 to about 10.5.

In some embodiments, the concentration of total tranexamic acid in the pharmaceutical composition is from about 1 mg/mL to about 80 mg/mL. In some embodiments, the concentration of total tranexamic acid in the pharmaceutical composition is from about 2.5 mg/mL to about 40 mg/mL. In some embodiments, the concentration of total tranexamic acid in the pharmaceutical composition is from about 5 mg/mL to about 25 mg/mL. In some embodiments, the concentration of total tranexamic acid in the pharmaceutical composition is from about 10 mg/mL to about 20 mg/mL.

In some embodiments, the unencapsulated tranexamic acid is about 1% to about 80% of the total amount of tranexamic acid in the pharmaceutical composition. In some embodiments, the unencapsulated tranexamic acid is about 20% to about 70% of the total amount of tranexamic acid in the pharmaceutical composition. In some embodiments, the unencapsulated tranexamic acid is about 30% to about 60% of the total amount of tranexamic acid in the pharmaceutical composition. In some embodiments, the unencapsulated tranexamic acid is about 50% of the total amount of tranexamic acid in the pharmaceutical composition. In some embodiments, the unencapsulated tranexamic acid is less than about 10% of the total tranexamic acid in the pharmaceutical composition.

In some embodiments, the ratio of unencapsulated (free) tranexamic acid to encapsulated tranexamic acid is between 1:10 to 10:1. In some embodiments, the ratio of unencapsulated (free) tranexamic acid to encapsulated tranexamic acid is 1:10. In some embodiments, the ratio of unencapsulated (free) tranexamic acid to encapsulated tranexamic acid is 1:9. In some embodiments, the ratio of unencapsulated (free) tranexamic acid to encapsulated tranexamic acid is 1:8. In some embodiments, the ratio of unencapsulated (free) tranexamic acid to encapsulated tranexamic acid is 1:7. In some embodiments, the ratio of unencapsulated (free) tranexamic acid to encapsulated tranexamic acid is 1:6. In some embodiments, the ratio of unencapsulated (free) tranexamic acid to encapsulated tranexamic acid is 1:5. In some embodiments, the ratio of unencapsulated (free) tranexamic acid to encapsulated tranexamic acid is 1:4. In some embodiments, the ratio of unencapsulated (free) tranexamic acid to encapsulated tranexamic acid is 1:3. In some embodiments, the ratio of unencapsulated (free) tranexamic acid to encapsulated tranexamic acid is 1:2. In some embodiments, the ratio of unencapsulated (free) tranexamic acid to encapsulated tranexamic acid is 1:1.

In some embodiments, the ratio of unencapsulated (free) tranexamic acid to encapsulated tranexamic acid is 2:1. In some embodiments, the ratio of unencapsulated (free) tranexamic acid to encapsulated tranexamic acid is 3:1. In some embodiments, the ratio of unencapsulated (free) tranexamic acid to encapsulated tranexamic acid is 4:1. In some embodiments, the ratio of unencapsulated (free) tranexamic acid to encapsulated tranexamic acid is 5:1. In some embodiments, the ratio of unencapsulated (free) tranexamic acid to encapsulated tranexamic acid is 6:1. In some embodiments, the ratio of unencapsulated (free) tranexamic acid to encapsulated tranexamic acid is 7:1. In some embodiments, the ratio of unencapsulated (free) tranexamic acid to encapsulated tranexamic acid is 8:1. In some embodiments, the ratio of unencapsulated (free) tranexamic acid to encapsulated tranexamic acid is 9:1. In some embodiments, the ratio of unencapsulated (free) tranexamic acid to encapsulated tranexamic acid is 10:1.

Some embodiments provide a pharmaceutical composition comprising tranexamic acid containing multivesicular liposomes prepared by the process described herein.

Any of the features of an embodiment is applicable to all embodiments identified herein. Moreover, any of the features of an embodiment is independently combinable, partly or wholly with other embodiments described herein in any way, e.g., one, two, or three or more embodiments may be combinable in whole or in part. Further, any of the features of an embodiment may be made optional to other embodiments. Any embodiment of a method can comprise another embodiment of a compound, and any embodiment of a compound can be configured to perform a method of another embodiment.

### DETAILED DESCRIPTION OF EMBODIMENTS

The present embodiments provide pharmaceutical formulations comprising multivesicular liposomes (MVLs) containing tranexamic acid (TXA) which minimize the side effects of TXA while maintaining or improving efficacy and lengthening the duration of the effect. The present embodiments also provide pharmaceutical formulations comprising TXA encapsulated in the MVLs and unencapsulated TXA. The term "unencapsulated" as used herein, means that the biologically active ingredient (e.g., tranexamic acid) is outside the MVL particles, for example, in the suspending solution. The unencapsulated TXA provides immediate efficacy of TXA while the use of TXA encapsulated MVLs formulations in the instant embodiments results in the release of TXA for an extended period. The processes of preparing the DEPO-TXAs and the methods of using the DEPO-TXA formulations for treating, ameliorating, or preventing blood loss are also disclosed herewith.

As used herein, the term "DEPO-TXA" refers to a multivesicular liposome formulation encapsulating tranexamic acid. DEPO-TXA also includes free tranexamic acid in the aqueous suspending solution of the MVLs. Preferably, the concentration of the free TXA in the DEPO-TXA aqueous suspending solution is approximately equal to the concentration of TXA encapsulated in the multivesicular liposomes.

As used herein, the term "free TXA" refers to a pharmaceutical formulation comprising unencapsulated tranexamic acid, for example, a saline solution containing TXA, or aqueous TXA.

As used herein "TXA-MVL" refers to a pharmaceutical formulation comprising a multivesicular liposome formulation encapsulating tranexamic acid with less than 10%, 9%, 8%, 7%, 6%, 5%, 4%, 3%, 2%, 1%, 0.5%, 0.2% or 0.1% free TXA, or a range defined by any of the two preceding values.

Unless defined otherwise, all technical and scientific terms used herein have the same meaning as is commonly understood by one of ordinary skill in the art. In the event that there are a plurality of definitions for a term herein, those in this section prevail unless stated otherwise. As used in the specification and the appended claims, the singular forms "a," "an" and "the" include plural referents unless the context clearly dictates otherwise. Unless otherwise indicated, conventional methods of mass spectroscopy, NMR, HPLC, protein chemistry, biochemistry, recombinant DNA techniques and pharmacology are employed. The use of "or" or "and" means "and/or" unless stated otherwise. Furthermore, use of the term "including" as well as other forms, such as "include", "includes," and "included," is not limiting. As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least." When used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

The section headings used herein are for organizational purposes only and are not to be construed as limiting the subject matter described.

### Multivesicular Liposomes Formulations

The instant embodiments are directed to MVLs containing TXA. MVLs, reported in Kim et al. (Biochim. Biophys. Acta, 728:339-348, 1983), are a group of unique forms of synthetic membrane vesicles that are different from other lipid-based delivery systems such as unilamellar liposomes (Huang, Biochemistry, 8:334-352, 1969; Kim, et al., Biochim. Biophys. Acta, 646:1-10, 1981) and multilamellar liposomes (Bangham, et al., J Mol. Bio., 13:238-252, 1965). The main structural difference between multivesicular liposomes and unilamellar liposomes (also known as unilamellar vesicles), is that multivesicular liposomes contain multiple aqueous chambers per particle. The main structural difference between multivesicular liposomes and multilamellar liposomes (also known as multilamellar vesicles), is that in multivesicular liposomes the multiple aqueous chambers are non-concentric. Multivesicular liposomes generally have between 100 to 1 million chambers per particle and all the internal chambers are interconnected by shared lipid-bilayer walls that separate the chambers. The structural differences between unilamellar, multilamellar, and multivesicular liposomes are illustrated in Sankaram et al., U.S. Pat. Nos. 5,766,627 and 6,132,766.

The structural and functional characteristics of multivesicular liposomes are not directly predictable from current knowledge of unilamellar vesicles and multilamellar vesicles. Multivesicular liposomes have a very distinctive internal morphology, which may arise as a result of the special method employed in the manufacture. Topologically, multivesicular liposomes are defined as having multiple non-concentric chambers within each particle, resembling a "foam-like" or "honeycomb-like" matrix; whereas multilamellar vesicles contain multiple concentric chambers within each liposome particle, resembling the "layers of an onion."

The presence of internal membranes distributed as a network throughout multivesicular liposomes may serve to confer increased mechanical strength to the vesicle. The particles themselves can occupy a very large proportion of the total formulation volume. The packed particle volume (PPV) of MVLs which is measured in a manner analogous to a hematocrit, representing the volume of the formulation that the particles make up and can approach as high as 80%. Typically the PPV is about 50%. At 50% PPV, the multivesicular liposome formulation typically consists of less than 5% w/w lipid. Thus, the encapsulated volume is approximately 50% while having a relatively low lipid concentration. The multivesicular nature of multivesicular liposomes also indicates that, unlike for unilamellar vesicles, a single breach in the external membrane of multivesicular vesicles will not result in total release of the internal aqueous contents.

Thus, multivesicular liposomes formulations consist of microscopic, spherical particles composed of numerous nonconcentric aqueous chambers encapsulating TXA to be delivered. The individual chambers are separated by lipid bilayer membranes composed of synthetic versions of naturally occurring lipids, resulting in a delivery vehicle that is both biocompatible and biodegradable. The instant DEPO-TXA formulations provide either local site or systemic sustained delivery, and can be administered by a number of routes including topical and various parenteral applications, such as subcutaneous injection, muscle injection, wound infiltration or wound instillation.

Some embodiments of the present application are related to pharmaceutical compositions comprising tranexamic acid encapsulated multivesicular liposomes ("MVLs"), the multivesicular liposomes comprising tranexamic acid ("TXA"), a lipid component comprising at least one amphipathic lipid and at least one neutral lipid; and one or more pH modifying agents. In some embodiments, the MVLs can optionally comprise a second therapeutic agent. In some other embodiments, TXA is the only therapeutic agent in the MVLs.

Some embodiments of the present application are related to pharmaceutical compositions comprising: multivesicular liposomes encapsulating tranexamic acid comprising tranexamic acid, a lipid component comprising at least one amphipathic lipid and at least one neutral lipid, and one or more pH modifying agents; and unencapsulated tranexamic acid.

In some embodiments, the MVLs further comprise cholesterol and/or a plant sterol.

In some embodiments, the amphipathic lipid comprises phosphatidylcholine, or phosphatidylglycerol or salts thereof, or combinations thereof. In some such embodiments, the phosphatidylglycerol is DPPG. In some such embodiments, the phosphatidylcholine is selected from DEPC or DOPC, or a combination thereof. In some other embodiments, the MVLs are DEPC-free. In some further embodiments, the MVLs are substantially free of phosphatidylcholines.

In some embodiments, the neutral lipid comprises triglyceride, propylene glycol ester, ethylene glycol ester, or squalene, or combinations thereof. In some embodiments, the neutral lipid comprises triglyceride. In some such embodiments, the triglyceride is selected from triolein or tricaprylin, or a combination thereof.

### pH Modifying Agents

In some embodiments, the pH modifying agents are selected from one or more organic acids, organic bases, inorganic acids, or inorganic bases, or combinations thereof. Suitable inorganic acids (also known as mineral acids) that can be used in the present application include, but are not limited to hydrochloric acid (HCl), sulfuric acid (H₂SO₄), phosphoric acid (H₃PO₄), nitric acid (HNO₃), etc. Suitable organic acids that can be used in the present application include, but are not limited to acetic acid, aspartic acid, citric acid, formic acid, glutamic acid, glucoronic acid, lactic acid, malic acid, tartaric acid, etc. Suitable organic bases that can be used in the present application include, but are not limited to histidine, arginine, lysine, tromethamine (Tris), etc. Suitable inorganic bases that can be used in the present application include, but are not limited to sodium hydroxide, calcium hydroxide, magnesium hydroxide, potassium hydroxide, etc. In some embodiments, the pH modifying agents are selected from inorganic acids, or organic bases, or combinations thereof. In some other embodiments, the pH modifying agents are selected from organic acids, or organic bases, or combinations thereof. In some such embodiments, the inorganic acid is selected from hydrochloric acid or phosphoric acid. In some such embodiments, the organic acid is selected from tartaric acid, or glutamic acid, or a combination thereof. In some embodiments, the organic base is selected from histidine, arginine, tromethamine or lysine, or combinations thereof. In one embodiment, the pH modifying agents of the MVLs are hydrochloric acid and lysine. In another embodiment, the pH modifying agents of the MVLs are phosphoric acid and lysine. In another embodiment, the pH modifying agents of the MVLs are tartaric acid and lysine. In yet another embodiment, the pH modifying agents of the MVLs are histidine and lysine. In yet another embodiment, the pH modifying agents of the MVLs are tromethamine and lysine.

In some embodiments, the MVLs have an external pH range of about 4.0 to about 9.0. In some such embodiments, the external pH range is from about 4.5 to about 8.5. In some embodiments, the MVLs have an internal pH range from about 3.0 to about 9.0. In some such embodiments, the internal pH range is from about 3.5 to about 5.5. In some further embodiments, the internal pH is about 5.5.

### Osmotic Agents and Density Modifying Agents

In some embodiments, the MVLs further comprise one or more pH modifying agents and/or density modifying agents. The osmotic agent used in the present application provides desired osmolality in the preparation of the first aqueous component and the second aqueous component of the MVLs. Non-limiting exemplary osmotic agents suitable for the MVL formulation of the present application include monosaccharides (e.g., glucose, and the like), disaccharides (e.g., sucrose and the like), and polysaccharide or polyols (e.g., sorbitol, mannitol, Dextran, and the like). In some other embodiments, the osmotic agents are selected from Dextran 40, sucrose, sorbitol, or combinations thereof.

In some embodiments, the osmotic agent can also act as a density modifying agent. For example, Dextran 40 can act as a density modifying agent to maximize the density of the MVLs with minimum change of osmolality. Other non-limiting examples of density modifying agents that are suitable for the present application include polysaccharides, poloxamers, polyethyleneglycols, carboxymethylcellulose, polyvinylpyrrolidone (PVP), polyvinylpolypyrrolidone (PVPP), etc.

In some embodiments, a pH modifying agent can also act as an osmotic agent. In one particular embodiment, TXA is also used as an osmotic agent.

In some embodiments, the concentration of total tranexamic acid in the pharmaceutical composition is from about 1 mg/mL to about 100 mg/mL. In some embodiments, the concentration of total tranexamic acid in the pharmaceutical composition is from about 1 mg/mL to about 80 mg/mL. In some embodiments the concentration of total tranexamic acid in the pharmaceutical composition is from about 2.5 mg/mL to about 40 mg/mL. In some embodiments, the concentration of total tranexamic acid in the pharmaceutical composition is from about 5 mg/mL to about 25 mg/mL. In some further embodiments, the concentration of total tranexamic acid in the pharmaceutical composition is from about 10 mg/mL to about 20 mg/mL. In yet still some embodiments, the concentration of total tranexamic acid in the pharmaceutical composition is from about 15 mg/mL to about 20 mg/mL.

In some embodiments, the unencapsulated tranexamic acid is about 10% to about 80% of the total amount of tranexamic acid in the pharmaceutical composition. In some such embodiments, the unencapsulated tranexamic acid is about 20% to about 70% of the total amount of tranexamic acid in the pharmaceutical composition. In some such embodiments, the unencapsulated tranexamic acid is about 30% to about 60% of the total amount of tranexamic acid in the pharmaceutical composition. In some further embodiments, the unencapsulated tranexamic acid is about 50% of the total amount of tranexamic acid in the pharmaceutical composition. In some other embodiments, the unencapsulated tranexamic acid is less than about 10% of the total tranexamic acid in the pharmaceutical composition.

In some embodiments, the DEPO-TXA formulation is administered one, two, three, four, or more times per day. The DEPO-TXA formulation can also be administered less than once per day, for example once every 2, 3, 4, 5, 6, 7, 8, 9, 10, 11, 12, 13, or 14 days, or every 1 or 2 weeks, or a range defined by any two of the preceding values. In some embodiments, the number of administrations per day is constant (e.g., one time per day). In other embodiments, the number of administrations is variable. The number of administrations may change depending on effectiveness of the dose, observed side effects, desire to titrate up to a desired dose, external factors (*e.g.,* a change in another medication), or the length of time that the dosage form has been administered.

In some embodiments, the DEPO-TXA formulation is administered in a dose ranging from about 10 mg/kg to about 500 mg/kg. In some further embodiments, the formulation is administered in a dose ranging from about 20 mg/kg to about 250 mg/kg. In still some further embodiments, the formulation is administered in a dose ranging from about 40 mg/kg to about 125 mg/kg. In one embodiment, the DEPO-TXA formulation is administered in a dose of about 40 mg/kg. In another embodiment, the formulation is administered in a dose of about 120 mg/kg.

In some embodiments, the Cmax of TXA in the DEPO-TXA formulation described herein is from about 10 mg/L to about 200 mg/L, from about 20 mg/L to about 150 mg/L, from about 40 mg/L to about 100 mg/L. In some further embodiments, the Cmax of TXA in the DEPO-TXA formulation is from about 45 mg/L to about 140 mg/L.

### Cyclodextrins

In certain embodiments, cyclodextrins can also be used in the DEPO-TXAs. In some other embodiments, the pharmaceutical composition of the present application is cyclodextrin free.

Cyclodextrins are chiral, toroidal-shaped molecules formed by the action of the enzyme cyclodextrin transglycosylase on starch. These cyclic oligomers contain from 6 to 12 glucose units bonded through α-(1,4)-linkages. The three smallest homologs, α-cyclodextrin, β-cyclodextrin and γ-cyclodextrin are available commercially; larger homologs must be produced and isolated individually. The secondary 2- and 3-hydroxy groups line the mouth of the cyclodextrin cavity and have a staggered orientation. The primary 6-hydroxyls are at the opposite end of the molecule. The inside of the cyclodextrin cavity is relatively hydrophobic since all hydroxyls are directed toward the outside of the molecule.

Many different types of cyclodextrins can be useful in the compositions and methods of the present embodiments. Such cyclodextrins include, but are not limited to, (2,6-di-O-)ethyl-β-cyclodextrin, (2-carboxyethyl)-β-cyclodextrin sodium salt, (2-hydroxyethyl)-β-cyclodextrin, (2-hydroxypropyl)-α-cyclodextrin, sulfobutylether-β-cyclodextrin, (2-hydroxypropyl)-β-cyclodextrin, 6-monodeoxy-6-monoamino-β-cyclodextrin, 6-O-α-maltosyl-β-cyclodextrin, butyl-β-cyclodextrin, butyl-γ-cyclodextrin, carboxymethyl-β-cyclodextrin, methyl-β-cyclodextrin, succinyl-α-cyclodextrin, succinyl-β-cyclodextrin, triacetyl-β-cyclodextrin, α-cyclodextrin β-cyclodextrin, and γ-cyclodextrin.

In some embodiments, the MVL formulations of the present application optionally include a pharmaceutically acceptable carrier.

The DEPO-TXAs formulation of the present application is stable at 4°C for at least 1 week, 2 week or 4 months. The DEPO-TXA formulation of the present application is also stable at 37 °C for at least 2 days. The term "stable" as used herein, refers to the encapsulated TXA staying within the MVLs under certain environmental conditions for a period of time without excessively leaking out of MVLs in free form. In some embodiments, the DEPO-TXA formulations of the present application are stable at 4°C for 4 months with less than 6 percent, less than 5 percent, less than 4 percent, less than 3 percent, less than 2 percent or less than 1 percent TXA in free form. In some embodiments, the DEPO-TXA formulations of the present application are stable at 37 °C for 2 days with less than 50 percent, less than 40 percent, less than 35 percent, less than 30 percent, less than 25 percent, less than 20 percent, less than 15 percent, more preferably less than 10 percent, less than 5 percent TXA in free form.

### Methods of Manufacturing

Some other embodiments of the present application are related to processes for preparing multivesicular liposomes comprising tranexamic acid, said process comprising: preparing a first aqueous component comprising tranexamic acid and at least one pH modifying agent; preparing a lipid component comprising at least one organic solvent, at least one amphipathic lipid, and at least one neutral lipid; mixing said first aqueous component and said lipid component to form a water-in-oil emulsion, wherein at least one component comprises tranexamic acid; contacting said water-in-oil emulsion with a second aqueous component to form solvent-containing spherules; and removing the organic solvent from the solvent-containing spherules to form multivesicular liposomes. In some embodiments, the process further comprises an additional step of suspending the multivesicular liposomes in a solution that may comprise free tranexamic acid to form a pharmaceutical composition comprising both encapsulated and unencapsulated tranexamic acid.

Optionally, other components are included in the lipid phase. Among these are antioxidants, antimicrobial preservatives, and cholesterol or plant sterols. In some embodiments, the lipid component further comprises cholesterol and/or a plant sterol.

A "water-in-oil" type emulsion is formed from two immiscible phases, a lipid phase and a first aqueous phase. The lipid phase is made up of at least one amphipathic lipid and at least one neutral lipid in a volatile organic solvent, and optionally cholesterol and/or cholesterol derivatives. The term "amphipathic lipid" refers to molecules having a hydrophilic "head" group and a hydrophobic "tail" group and may have membrane-forming capability. As used herein, amphipathic lipids include those having a net negative charge, a net positive charge, and zwitterionic lipids (having no net charge at their isoelectric point). The term "neutral lipid" refers to oils or fats that have no vesicle-forming capabilities by themselves, and lack a charged or hydrophilic "head" group. Examples of neutral lipids include, but are not limited to, glycerol esters, glycol esters, tocopherol esters, sterol esters which lack a charged or hydrophilic "head" group, and alkanes and squalenes.

The amphipathic lipid is chosen from a wide range of lipids having a hydrophobic region and a hydrophilic region in the same molecule. Suitable amphipathic lipids include, but not limited to zwitterionic phospholipids, including phosphatidylcholines, phosphatidylethanolamines, sphingomyelins, lysophosphatidylcholines, and lysophosphatidylethanolamines; anionic amphipathic phospholipids such as phosphatidylglycerols, phosphatidylserines, phosphatidylinositols, phosphatidic acids, and cardiolipins; cationic amphipathic lipids such as acyl trimethylammonium propanes, diacyl dimethylammonium propanes, stearylamine, and the like. Preferred amphipathic lipids include dioleyl phosphatidyl choline (DOPC), dierucoyl phosphatidylcholine or 1,2-dierucoyl-sn-glycero-3-phosphocholine (DEPC), and dipalmitoylphosphatidylglycerol or 1,2-dipalmitoyl-sn-glycero-3-phospho-rac-(1-glycerol) (DPPG). In certain embodiments, amphipathic lipids used in the DEPO-TXA formulations include DOPC and/or DEPC in conjunction with DPPG.

Suitable neutral lipids include but are not limited to triglycerides, propylene glycol esters, ethylene glycol esters, and squalene. Non-limiting exemplary triglycerides useful in the instant formulations and methods are triolein (TO), tripalmitolein, trimyristolein, trilinolein, tributyrin, tricaproin, tricaprylin, and tricaprin. The fatty chains in the triglycerides useful in the present application can be all the same, or not all the same (mixed chain triglycerides), or all different. Propylene glycol esters can be mixed diesters of caprylic and capric acids.

In some embodiments, the amphipathic lipid comprises phosphatidylcholine, or phosphatidylglycerol or salts thereof, or combinations thereof. In some such embodiments, the phosphatidylglycerol is DPPG. In some such embodiments, the phosphatidylcholine is selected from DEPC or DOPC, or a combination thereof.

In some embodiments, the neutral lipid comprises triglyceride, propylene glycol ester, ethylene glycol ester, or squalene, or combinations thereof. In some embodiments, the neutral lipid comprises triglyceride. In some such embodiments, the triglyceride is selected from triolein or tricaprylin, or a combination thereof.

The concentrations of the amphipathic lipids, neutral lipids, and cholesterol present in the water-immiscible solvent used to make the MVLs typically range from 1-40 mM, 2-40 mM, and 0-60 mM, respectively. In some embodiments, the concentrations of the amphipathic lipids, neutral lipids, and cholesterol may range from about 20 mM to about 40 mM, about 5 mM to about 40 mM, and about 25 to about 40 mM, respectively. If a charged amphipathic lipid is included, it is generally present in a lower concentration than the zwitterionic lipid, when the zwitterionic lipid is present.

Many types of volatile organic solvents can be used in the present application, including ethers, esters, halogenated ethers, hydrocarbons, halohydrocarbons, or freon. For example, diethyl ether, chloroform, methylene chloride, tetrahydrofuran, ethyl acetate, and any combinations thereof are suitable for use in making the formulations. In some embodiments, methylene chloride is used. In some other embodiments, chloroform is used.

In certain embodiments, the first aqueous component comprises TXA and at least one pH modifying agent, optionally one or more osmotic agents described herein, or cyclodextrin(s). In some embodiments, the pH modifying agent of the first aqueous component is selected from an inorganic acid, an organic acid, an inorganic base, or an organic base, or combinations thereof. In some such embodiments, the pH modifying agent is selected from hydrochloric acid, phosphoric acid, or tartaric acid. In some other embodiments, the pH modifying agent is selected from histidine, arginine or tromethamine. In some embodiments, the osmotic agent is selected from a saccharide, such as sucrose. In some embodiments, the density modifying agent is selected from the Dextrans, for example Dextran-40. In some embodiments, the osmolality of the first aqueous component ranges from about 10 mOsm/kg to about 600 mOsm/kg. In some further embodiments, the osmolality of the first aqueous component ranges from about 285 mOsm/kg to about 335 mOsm/kg.

In some embodiments, the pH range of the first aqueous component is from about 2.0 to about 9.0. In some further embodiments, the pH range of the first aqueous component is from about 3.5 to about 5.5. In one embodiment, the pH range of the first aqueous component is about 5.5. In some further embodiments, the pH range of the first aqueous component is from about 4.3 to about 5.5. In some further embodiments, the pH range of the first aqueous component is from about 7.5 to about 9.0. In some further embodiments, the pH of the first aqueous component is about 7.7. The pH of the first aqueous component has an impact on the stability of the finished TXA encapsulated MVLs. In certain cases, it was observed that when the pH level was high in the first aqueous component, the encapsulated TXA was more likely to leak out of the MVLs. In contrast, lower pH level in the first aqueous component renders the finished product more stable at higher storing temperatures (for example, room temperature or 37°C). In some embodiments, the preferred pH range is from about 3.5 to about 5.5, more preferably from about 3.5 to about 4.4.

The lipid phase and first aqueous phase are mixed by mechanical turbulence, such as through use of rotating or vibrating blades, shaking, extrusion through baffled structures or porous pipes, or by ultrasound, or by the use of a three fluid nozzle (described in Schutt et al., U.S. Pub. No. 2011/0250264 A1) to produce a water-in-oil emulsion. The water-in-oil emulsion can then be dispersed into a second aqueous phase by means described above, to form solvent-containing spherules suspended in the second aqueous phase, a water-in-oil-in-water emulsion is formed. The term "solvent-containing spherules" refers to a microscopic spheroid droplet containing organic solvent, within which are suspended multiple smaller droplets of aqueous solution. The second aqueous phase can contain additional components such as one or more pH modifying agents, and one or more osmotic agents and combinations thereof. In some embodiments, the second aqueous component comprises lysine or histidine as a pH modifying agent. In some embodiments, the pH range of the second aqueous component is from about 3.5 to about 10.5. In some embodiments, the pH range of the second aqueous component is from about 7.5 to about 10.5. In some embodiments, the second aqueous component comprises sorbitol or sucrose as an osmotic agent. In one particular embodiment, TXA is also used as an osmotic agent. In some embodiments, the osmolality of the second aqueous component ranges from about 10 mOsm/kg to about 600 mOsm/kg. In some further embodiments, the osmolality of the second aqueous component ranges from about 270 mOsm/kg to about 350 mOsm/kg.

The volatile organic solvent is then removed from the spherules, for instance by surface evaporation from the suspension, sparging with a gas, or contacting with a gas in a spray chamber. When the solvent is substantially or completely evaporated, MVLs are formed. Gases which can be used for the evaporation include nitrogen, argon, helium, oxygen, hydrogen, and carbon dioxide, mixtures thereof, or clean compressed air. Alternately, the volatile solvent can be removed by sparging, rotary evaporation, diafiltration or with the use of solvent selective membranes, or contacting with a gas in a spray chamber.

As discussed above, TXA can be incorporated in the MVL by inclusion in the first aqueous component. TXA can also be incorporated in the MVLs by inclusion in the lipid phase or both the lipid and first aqueous component. The amount of TXA recovered in the instant MVLs was assayed by diluting the suspension of the DEPO-TXA 30 fold into 50% methanol in water, then injecting the resulting mixture into an HPLC (Hewlett-Packard Model 1100 with C-18 column; running solvent system: 51% MeOH; 49% aqueous buffer containing monobasic sodium phosphate (NaH₂PO₄), H₃PO₄, TEA and sodium dodecyl sulfate ("SDS"); pH=2.5) as described in the United States Pharmacopeia 37 (USP 37) assay for organic impurities with some minor modification. In some embodiments, the percent TXA yield is from about 40% to about 90% of the starting TXA amount, more preferably from about 50% to about 90%, more preferably from about 60% to about 90%.

Preparation of multivesicular liposomes is illustrated in Sankaram et al., U.S. Patent Nos. 5,766,627 and 6,132,766. Methods of making the instant MVL formulations can also be found in Hartounian et al. (WO99/25319) and Schutt et al. (U.S. Publication No. 2011/0250264 A1). Alternatively, TXA can be remotely loaded to the blank MVL particles. Such process is described in Garcia et al., U.S. Publication No. 2012/0114740.

### Methods of Administration

Some embodiments of the present application are related to methods of treating, ameliorating or preventing blood loss comprising administering a pharmaceutical composition comprising: tranexamic acid encapsulated multivesicular liposomes, the multivesicular liposomes comprising tranexamic acid, a lipid component comprising at least one amphipathic lipid and at least one neutral lipid, and one or more pH modifying agents; and unencapsulated tranexamic acid. In some embodiments, the administration is parenteral. In some other embodiments, the administration is topical. In some embodiments, the administration is both parenteral and topical. In some such embodiments, the parenteral administration is selected from subcutaneous injection, tissue injection, wound infiltration, or wound instillation. In some such embodiments, the topical administration comprises direct contact of said pharmaceutical composition with a cavity or a surface of the subject body that is in need of treatment, such as pouring the pharmaceutical composition onto an open wound.

As used herein, the term "subject" includes animals and humans. In a preferred embodiment, the subject is a human.

In any of the embodiments, the instant pharmaceutical compositions can be administered by bolus injection, e.g., subcutaneous bolus injection, intramuscular bolus injection, intradermal bolus injection and the like. In any of the embodiments, administration can be by infusion, e.g., subcutaneous infusion, intramuscular infusion, intradermal infusion, and the like. In any of the embodiments, administration can be direct wound infiltration by local injection into and/or around the wound margin or instillation into the incision, wound, or body cavity, or combinations thereof. The DEPO-TXA formulations can also be administered by other routes of administration including, but not limited to, topical, nasal, and systemic delivery such as IV.

Administration of the instant DEPO-TXA formulations is accomplished using standard methods and devices, e.g., pens, injector systems, needle and syringe, a subcutaneous injection port delivery system, catheters, and the like.

In some embodiments, the MVL formulations of the present application optionally include a pharmaceutically acceptable carrier. The term "pharmaceutically-acceptable carrier", as used herein, means one or more compatible solid or liquid filler diluents or encapsulating substances, which are suitable for administration to a mammal. The term "compatible", as used herein, means that the components of the composition are capable of being commingled with the subject compound, and with each other, in a manner such that there is no interaction, which would substantially reduce the pharmaceutical efficacy of the composition under ordinary use situations. Pharmaceutically-acceptable carriers must, of course, be of sufficiently high purity and sufficiently low toxicity to render them suitable for administration preferably to an animal, preferably mammal being treated.

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; malt; gelatin; talc; calcium sulfate; polyols such as propylene glycol, glycerine, sorbitol, mannitol, and polyethylene glycol; alginic acid; emulsifiers, such as the TWEENS; salts, such as sodium chloride; wetting agents, such sodium lauryl sulfate; coloring agents; flavoring agents; stabilizers; antioxidants; preservatives; pyrogen-free water; isotonic saline; and phosphate buffer solutions.

The choice of a pharmaceutically-acceptable carrier to be used in conjunction with the subject compound is basically determined by the way the compound is to be administered.

Effective injectable compositions containing these compounds may be in either suspension or solution form. In the solution form TXA is dissolved in a physiologically acceptable vehicle. Such vehicles comprise a suitable solvent, a tonicity agent such as sucrose or saline, preservatives such as benzyl alcohol, if needed, and buffers. Useful solvents include, for example, water and aqueous alcohols, glycols, and carbonate esters such as diethyl carbonate.

Injectable suspension compositions require a liquid suspending medium, with or without adjuvants, as a vehicle. The suspending medium can be, for example, aqueous solutions of sodium chloride, sucrose, polyvinylpyrrolidone, polyethylene glycol, or combinations of the above. In some embodiments, the suspension composition comprises a liquid suspending medium that is suitable for dissolving or solubilizing the unencapsulated tranexamic acid.

Suitable physiologically acceptable storage solution components are used to keep the compound suspended in suspension compositions. The storage solution components can be chosen from thickeners such as carboxymethylcellulose, polyvinylpyrrolidone, gelatin and the alginates. Many surfactants are also useful as suspending agents. The suspending medium could also contain lecithin, alkylphenol polyethylene oxide adducts, naphthalenesulfonates, alkylbenzenesulfonates, or the polyoxyethylene sorbitan esters. The MVLs storage suspension solution can contain additional additive(s).

Many substances which affect the hydrophilicity, density, and surface tension of the liquid suspending medium can assist in making injectable suspensions in individual cases. For example, silicone antifoams, sorbitol, and sugars can be useful suspending agents.

Some embodiments provide sustained release of TXA over 12 hours. Some embodiments provide sustained release of TXA over 24 hours. Some embodiments provide sustained release of TXA over 36 hours. Some embodiments provide sustained release of TXA over 48 hours. Some embodiments provide sustained release of TXA over 60 hours. Some embodiments provide sustained release of TXA over 72 hours. For example, Figures 5A and 5B illustrate the percent of total AUC for up to 72h after treatment on days 1 and 10, respectively. In the free TXA group, 94% of the administered TXA is cleared after 12 hours, whereas in the DepoTXA group, 25% of the dose remains at 12 hours, and is delivered over the next 60 hours. Similarly, Figures 6A and 6B illustrate the total amount of TXA delivered over 72 hours after administration on treatment days 1 and 10, respectively. Administration of TXA alone results in exposure to nearly the full TXA dose in less than 24 hours. In contrast, Depo-TXA provides, for example, exposure of 100 mg/kg TXA (from a 120 mg/kg dose) at 24 hours, demonstrating the sustained release profile of Depo-TXA.

### EXAMPLES

The following examples serve only to illustrate the invention and are not intended to limit the same.

### Example 1: DEPO-TXA Preparations

DEPO-TXA formulations were manufactured as follows: the therapeutic agent (TXA) is dissolved in the first aqueous solution comprising one or more pH modifying agents and one or more osmotic and/or density modifying agents, then the first aqueous solution was mixed with a lipid component comprising phospholipids and organic solvent with mechanical turbulence to form a water-in-oil emulsion; then the water-in-oil emulsion was dispersed into a second aqueous solution. A stream of nitrogen gas was passed over the mixture to evaporate the organic solvent. Saline solution was then added to the mixture and the MVLs were then isolated by centrifugation and washed.

Exemplary manufacturing condition and DEPO-TXA formulation assay results are summarized in Table 1 below. The process and apparatus for the bench scale preparation of the DEPO-TXA formulations described in entries 1-65 and 68-120 were disclosed in Sankaram et al., U.S. Patent No. 6,132,766 (for example, Example 1). Variations on the pH of the first aqueous component and the solutions and solvent are as stated in Table 1. In experiment entry 28, the MVLs were centrifuged and re-suspended in a solution containing 40 mg/mL free TXA, resulting in a suspension containing about 60% free TXA.

The process and apparatus for the spraying process of the DEPO-TXA formulations described in entries 66 and 67 were disclosed in Schutt et al., US 2011/0250264 A1, filed April 8, 2011 (for example, Example 4), with the exception that the solutions and solvent are as stated in Table 1, and the composition of the rinse solution is the same as the listed second aqueous solution in the table for those experiments.

TXA yield in the instant MVLs was assayed by diluting the suspension of the DEPO-TXAs 30 fold into 50% methanol in water, then injecting the resulting mixture into an HPLC (Hewlett-Packard Model 1100 with C-18 column; mobile phase solvent system: 51% MeOH; 49% aqueous buffer containing NaH₂PO₄, H₃PO₄, TEA and SDS; pH=2.5) as described below in the USP 37 assay for organic impurities with some minor modification.

**Table 1: Solution compositions and final product attributes for DEPO-TXA Formulations**

| Component | Grams | Final Vol. (mLs) | Final Conc. | Final units |
|---|---|---|---|---|
| monobasic sodium phosphate: | 11g | 600 | 1.833 | %, w/v |
| TEA: | 5mL | 600 | 0.833 | %, v/v |
| SDS: | 1.4g | 600 | 0.233 | %, w/v |
| H₃PO₄: | to pH 2.5 | 600 | na | na |

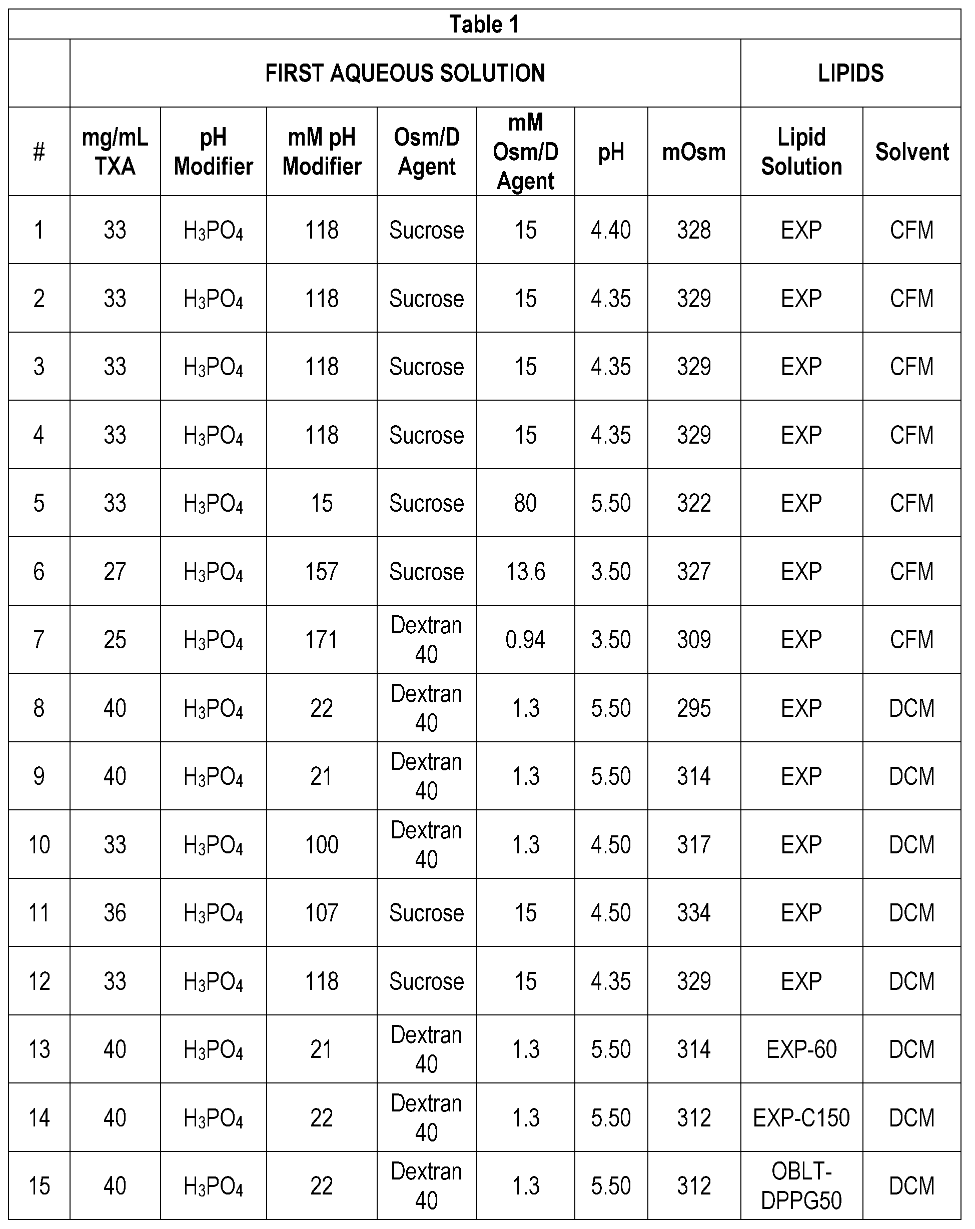

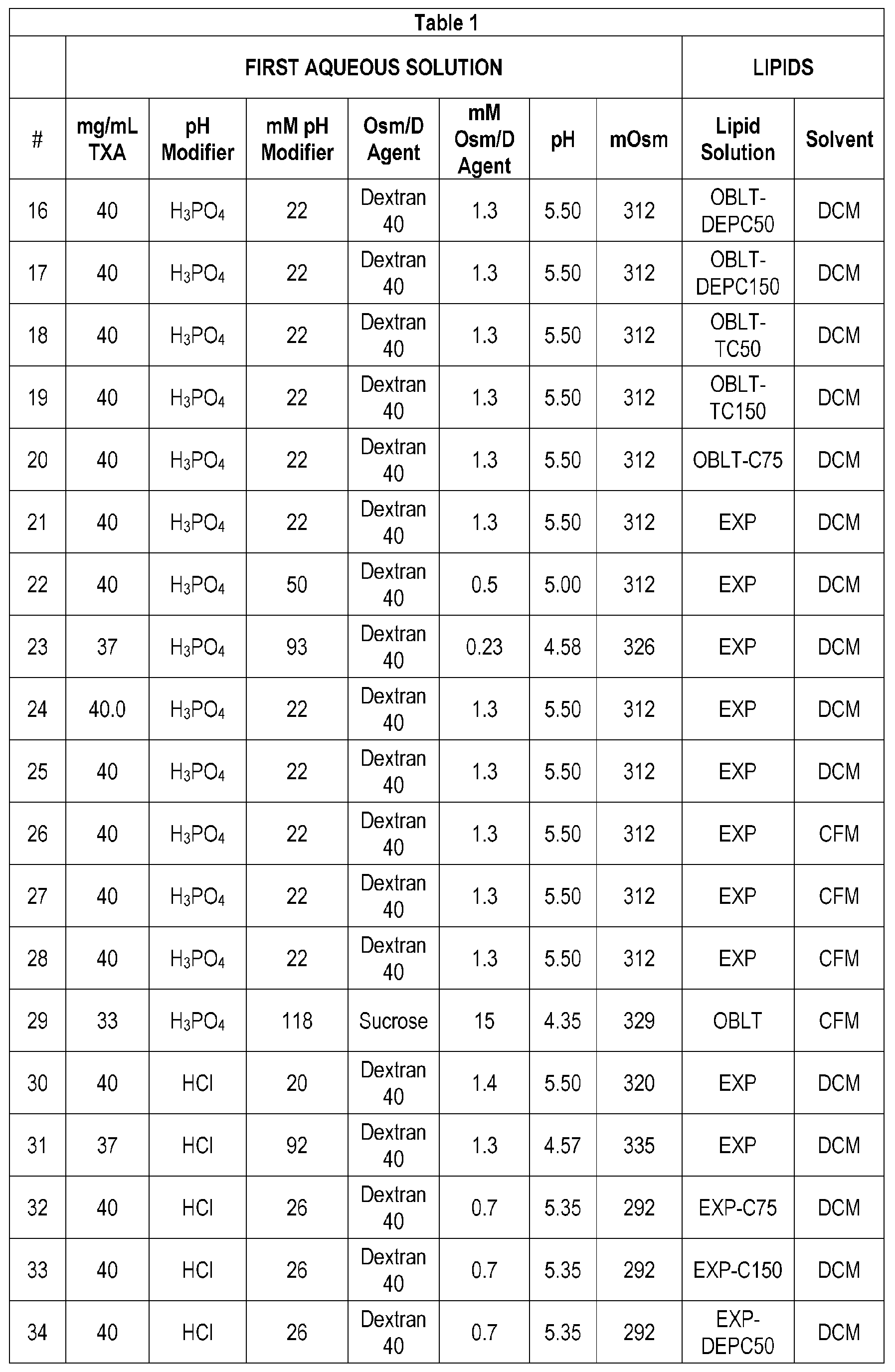

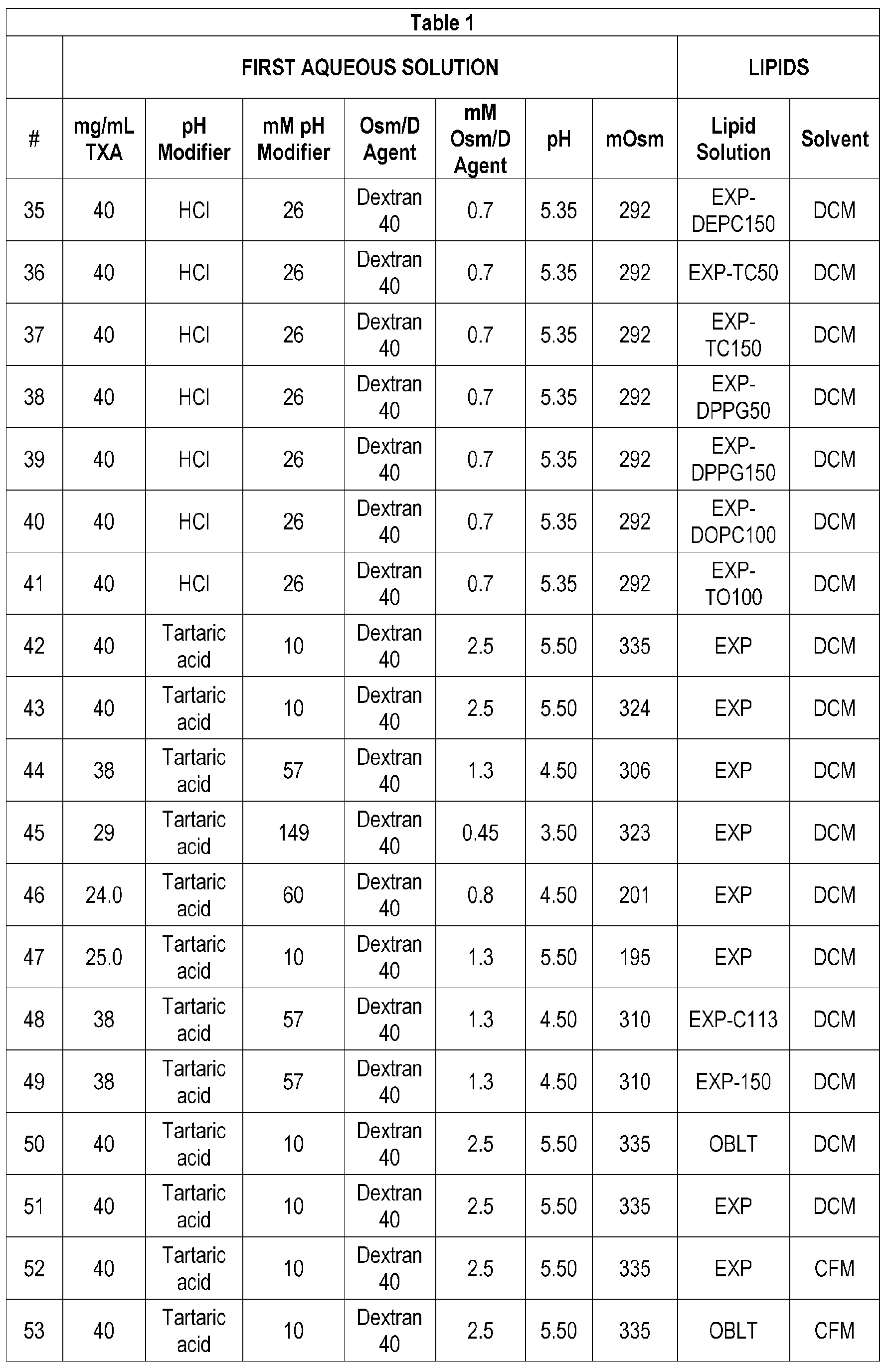

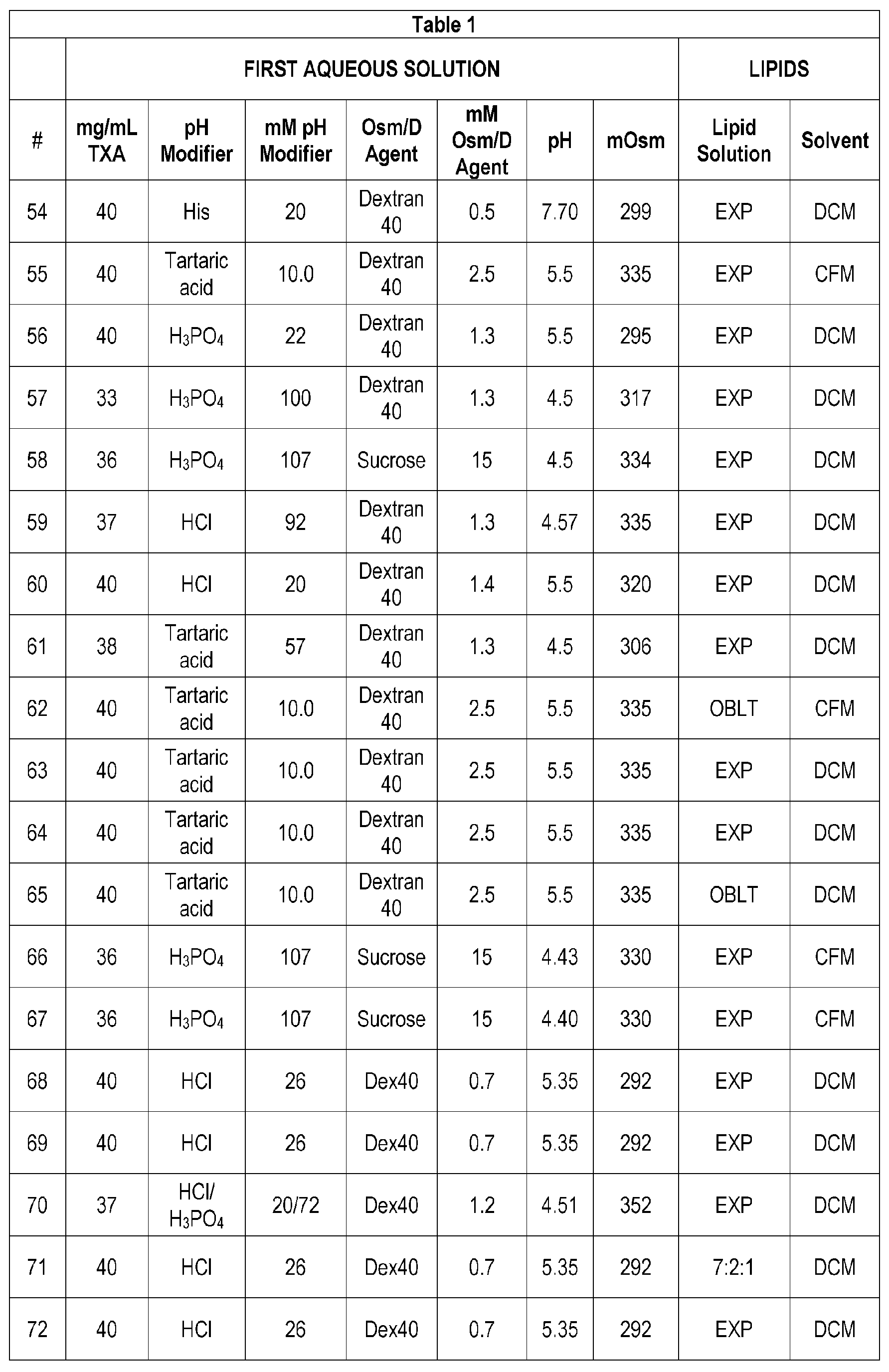

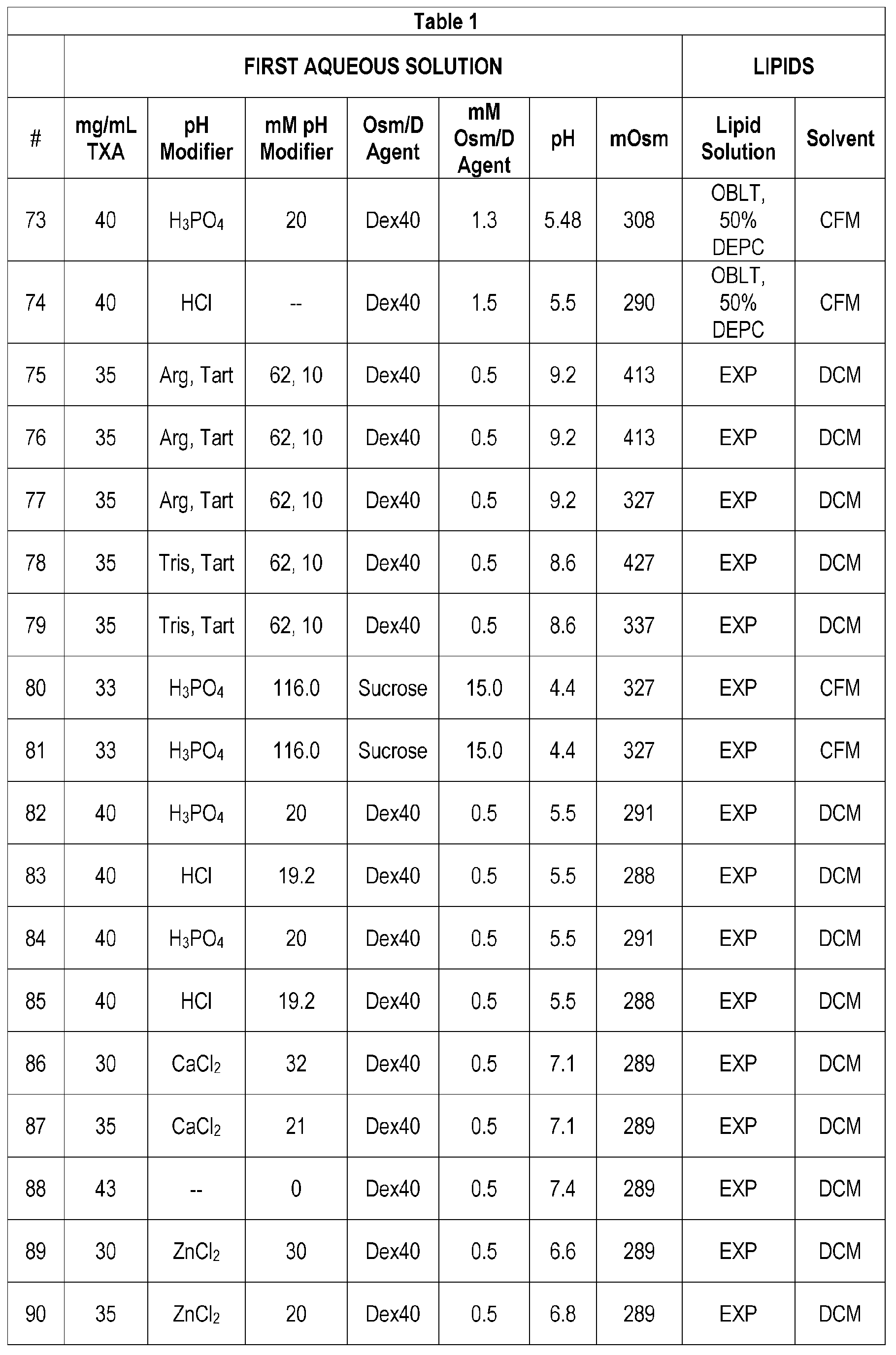

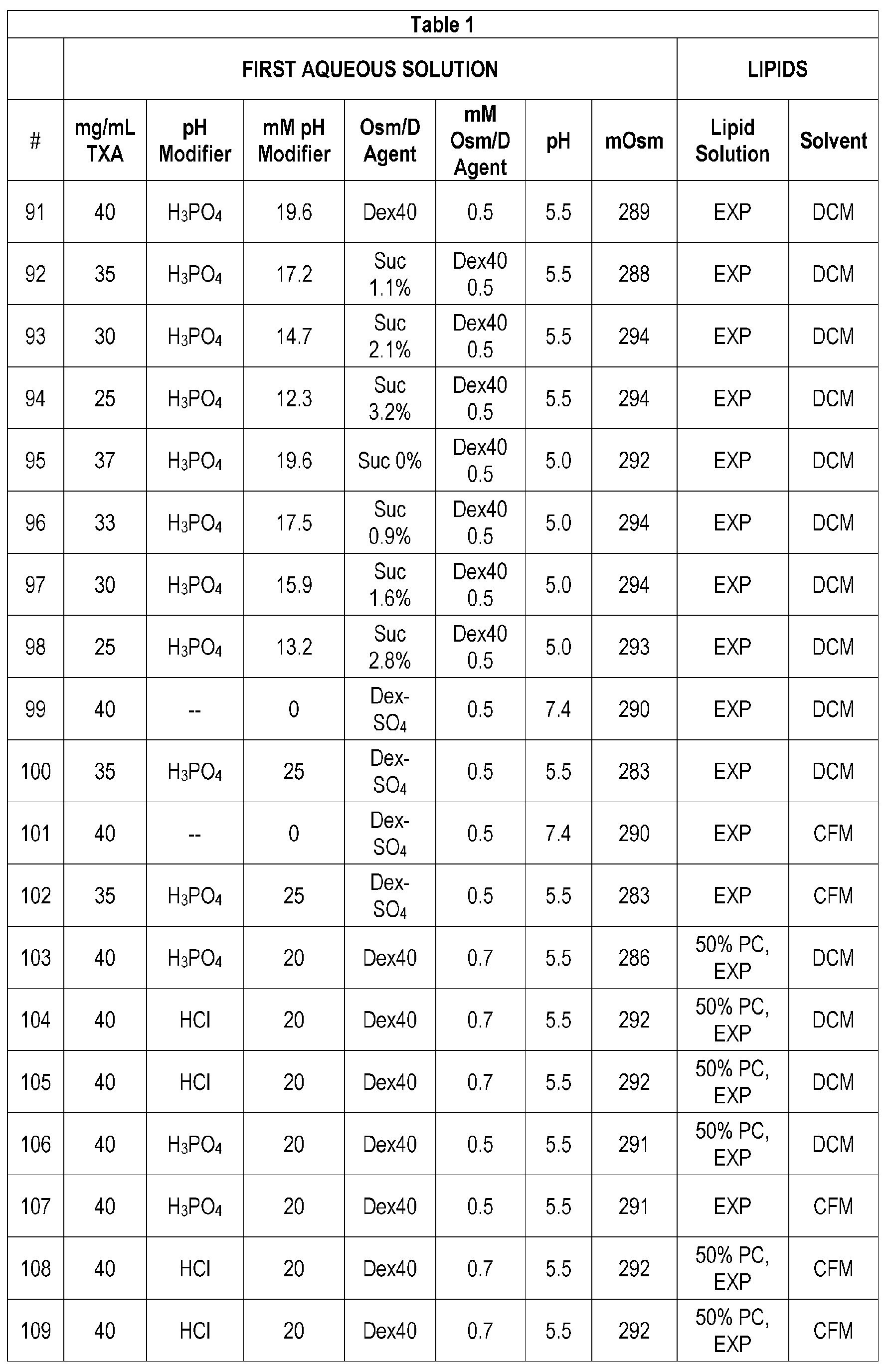

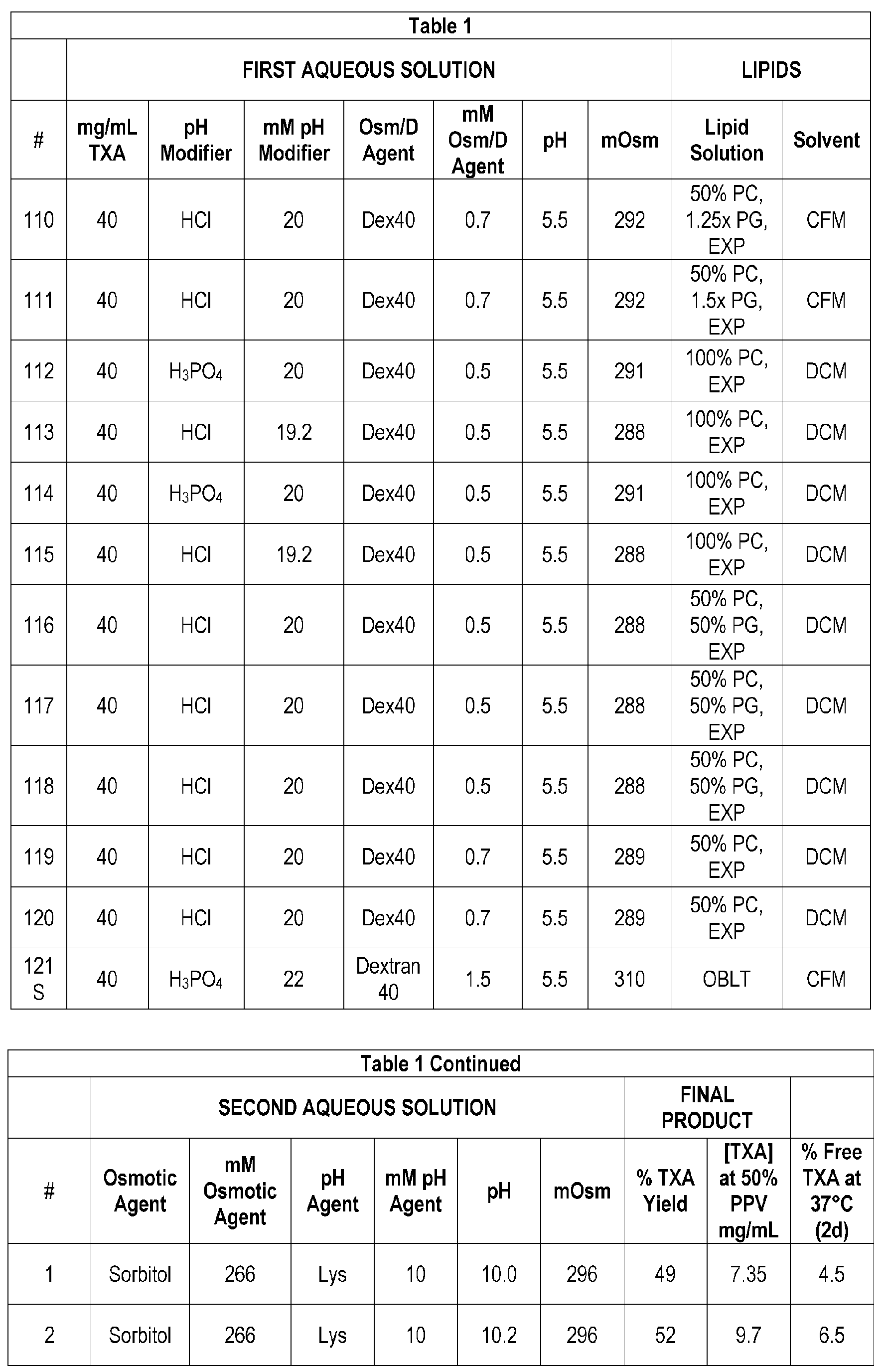

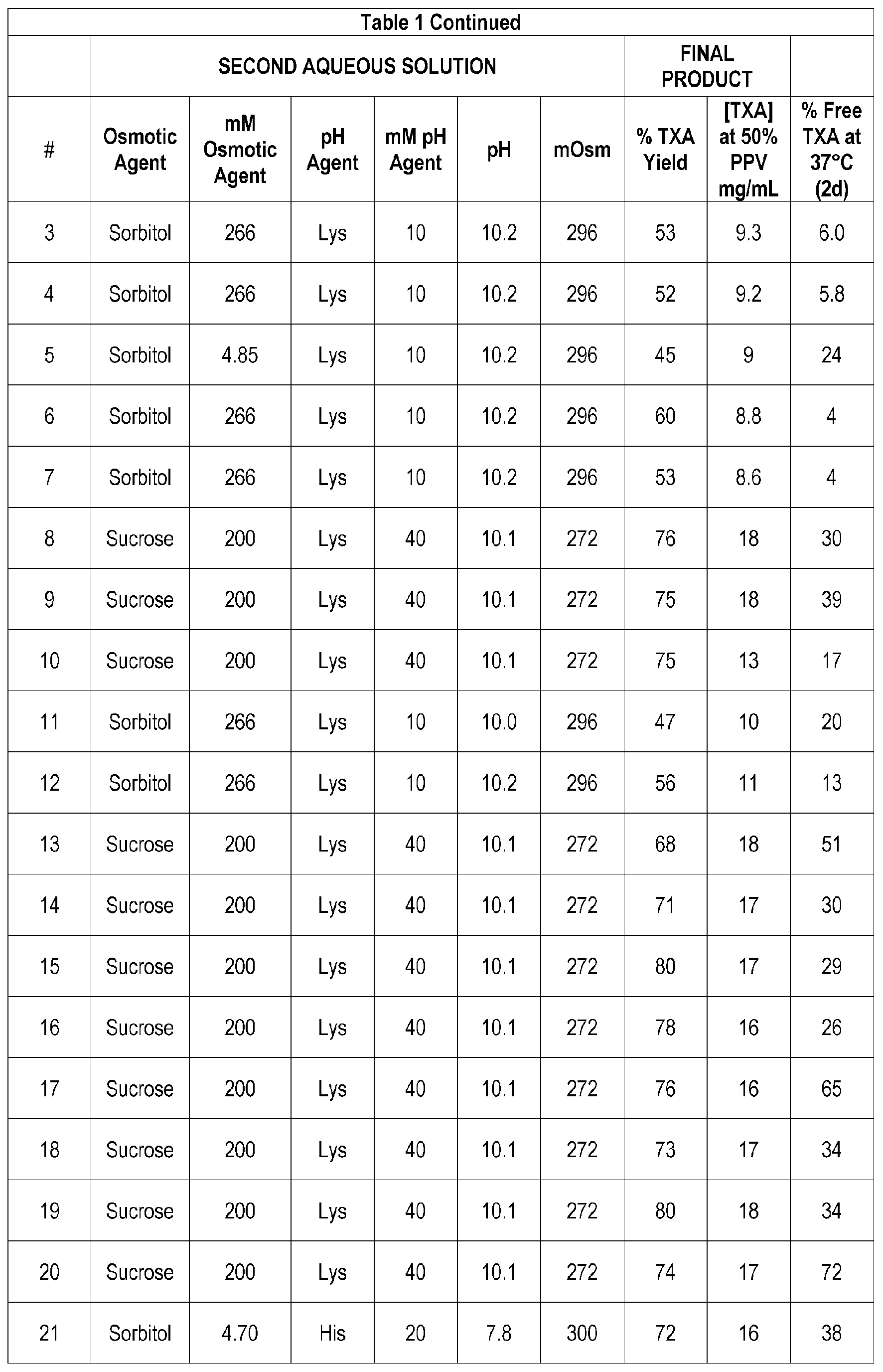

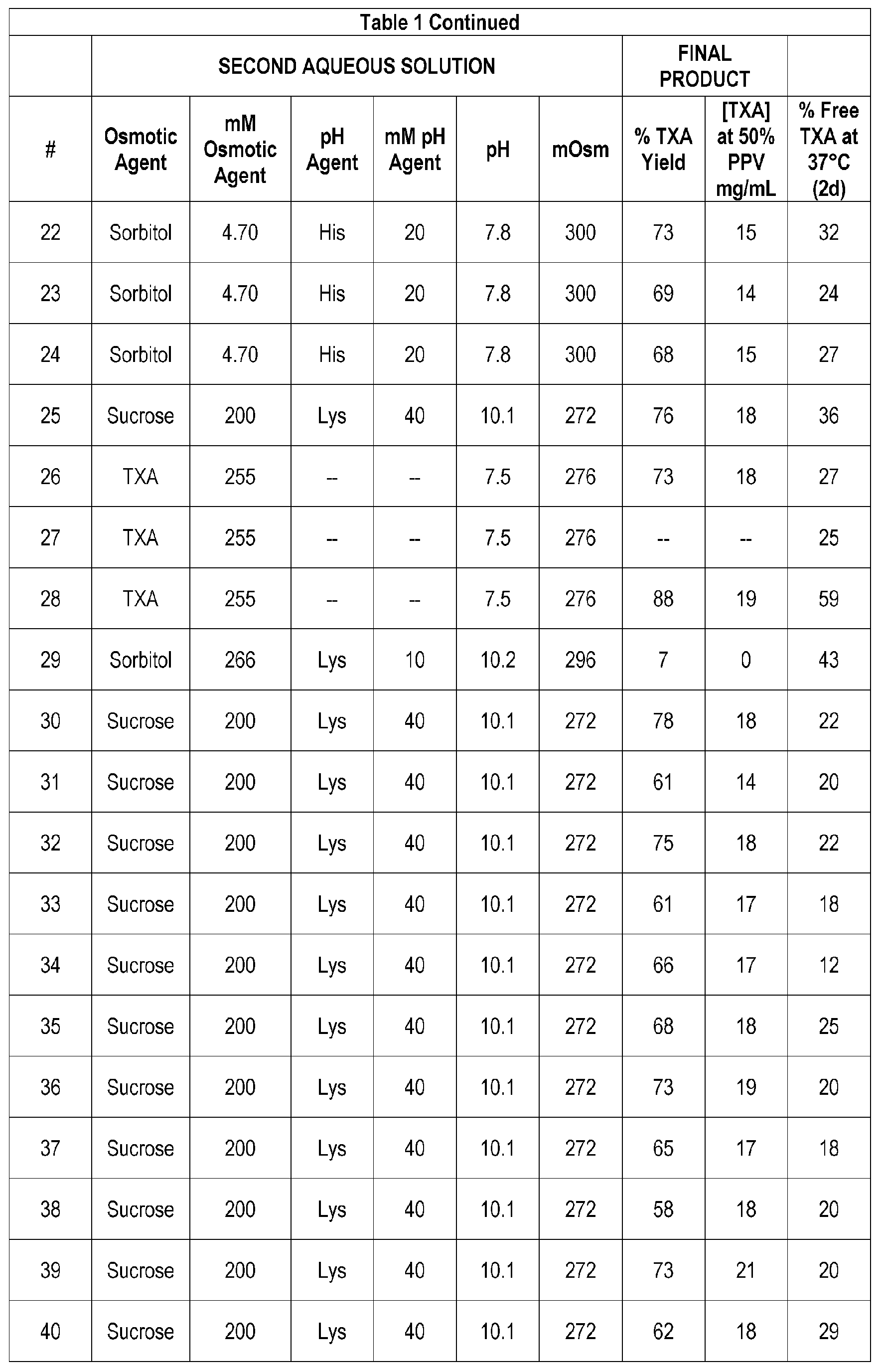

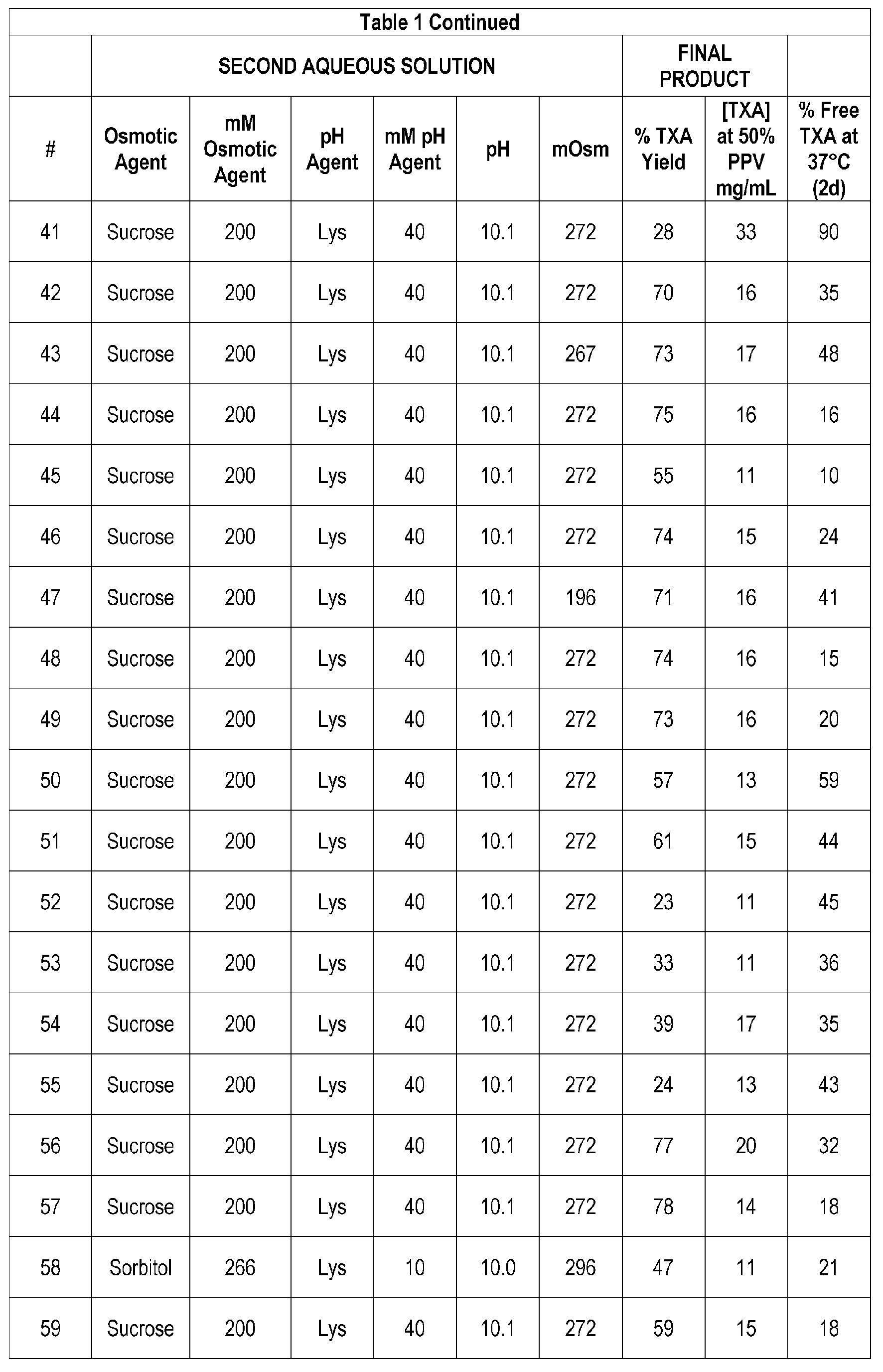

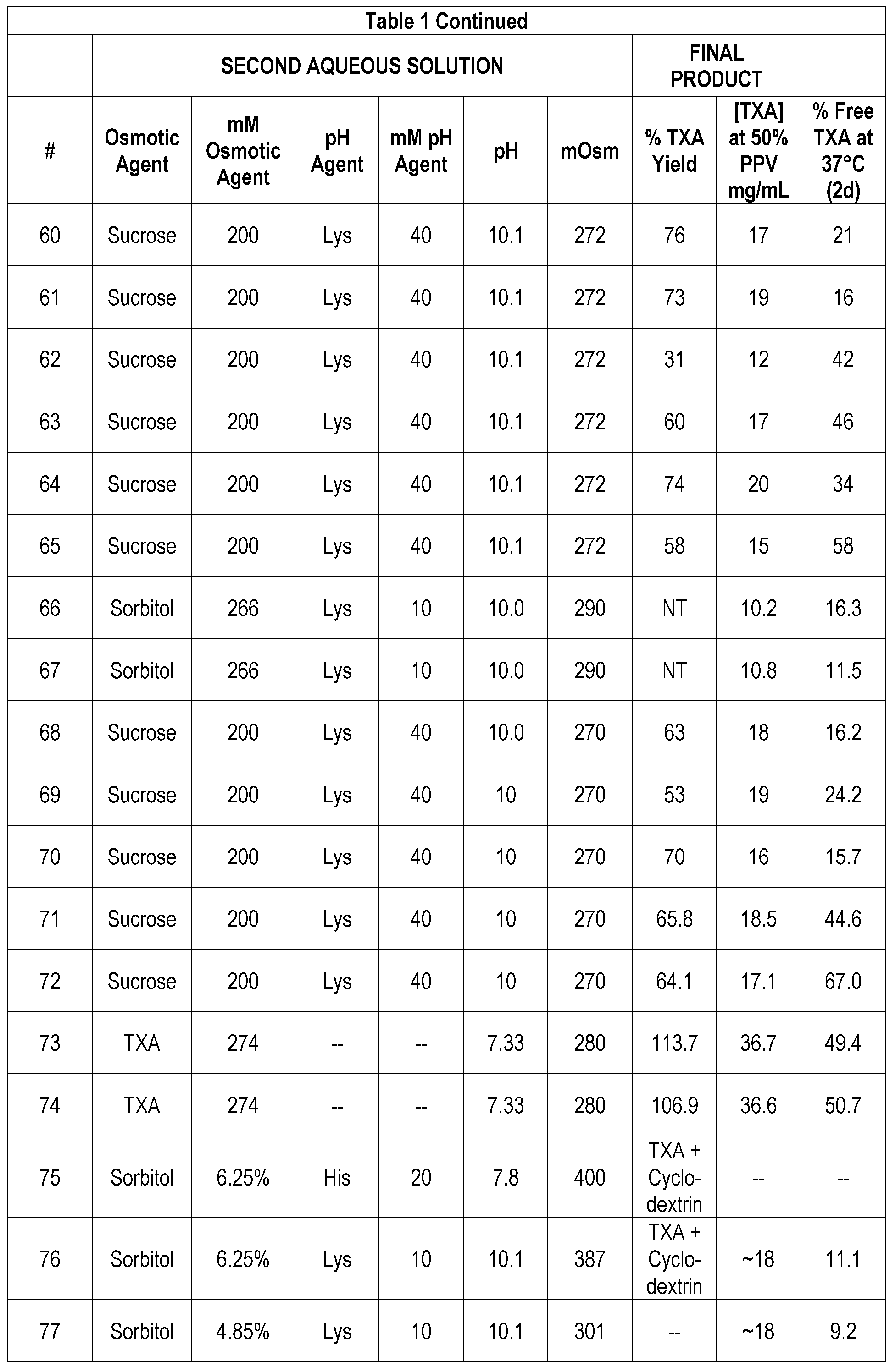

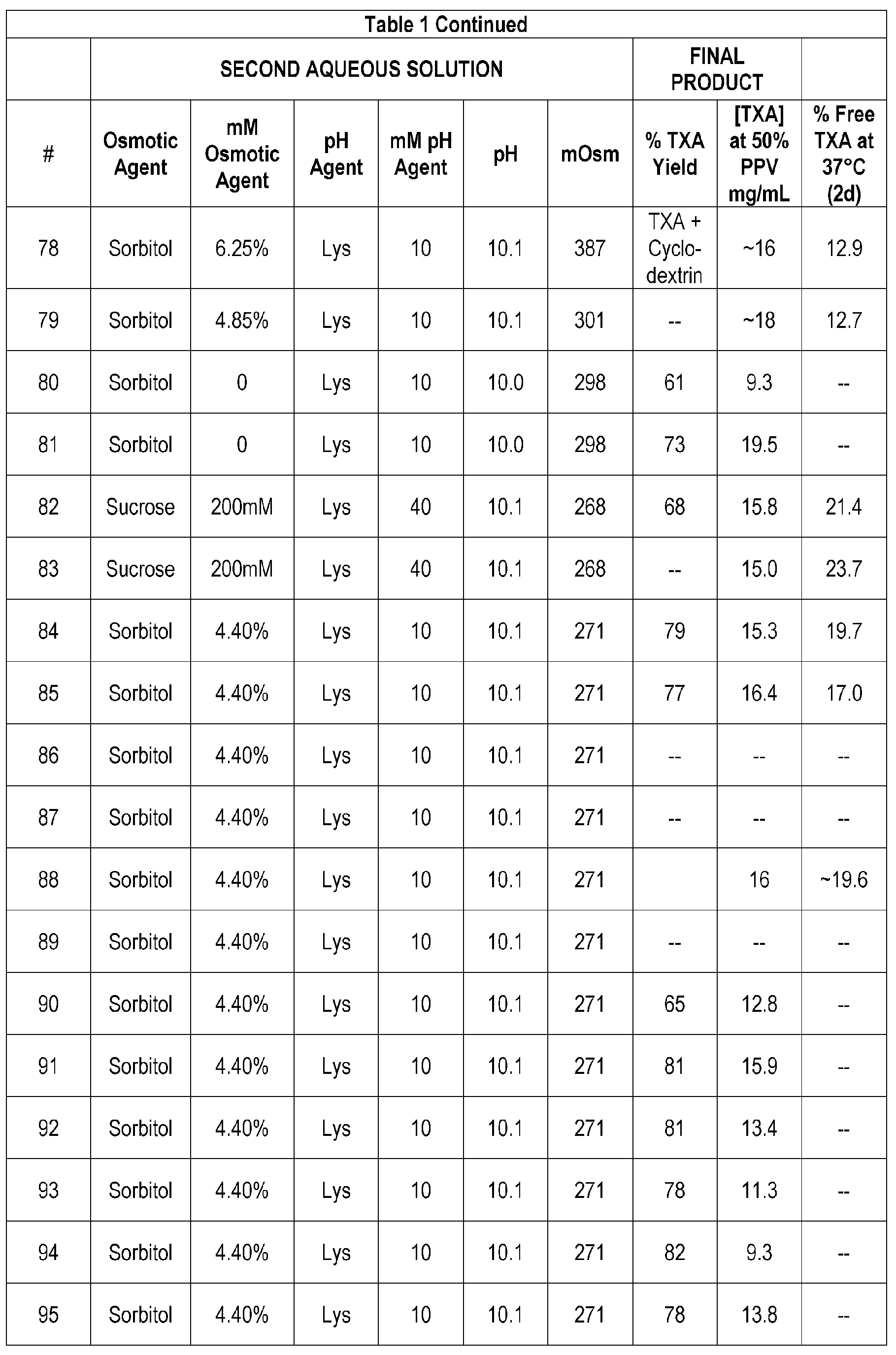

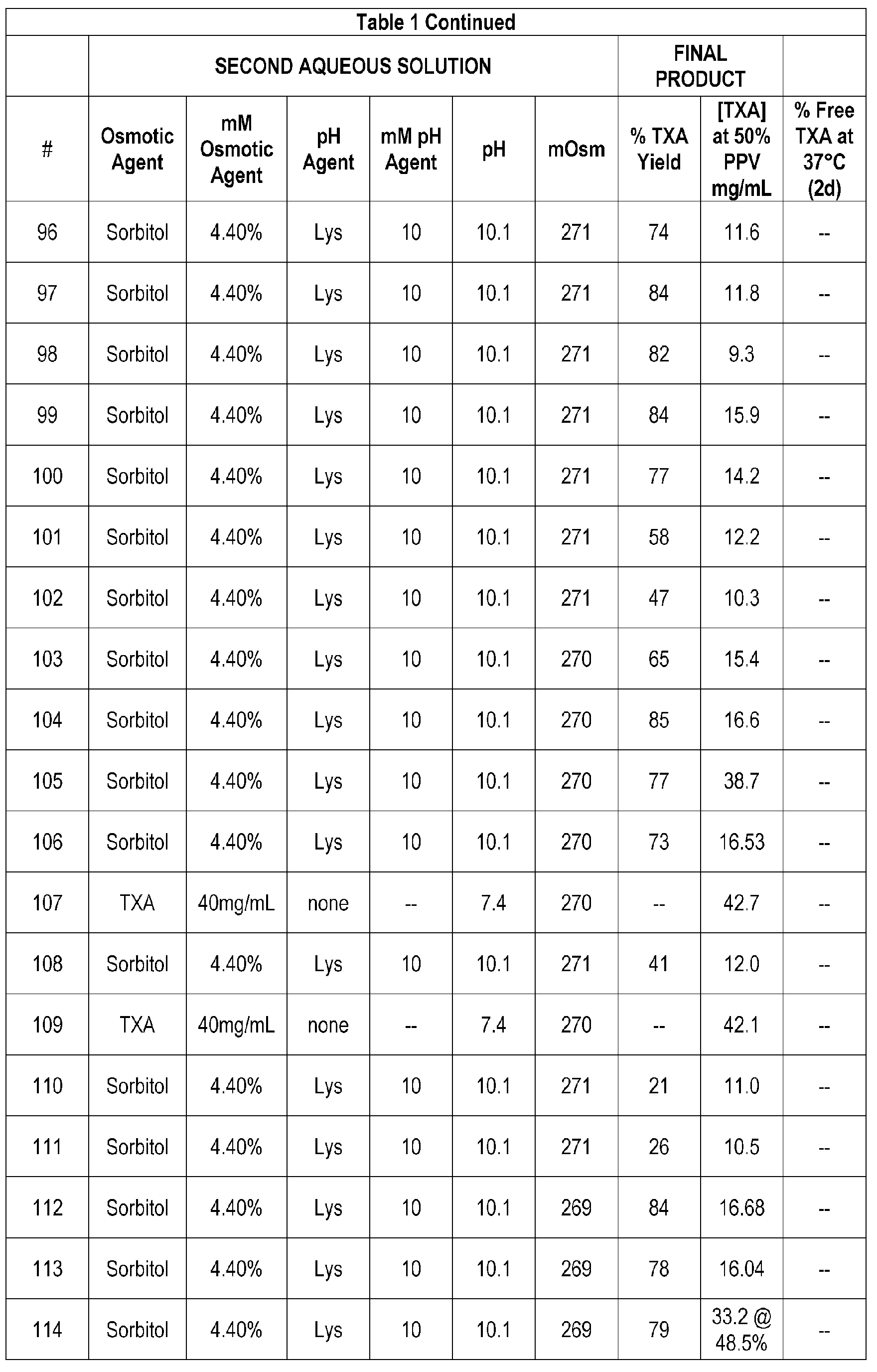

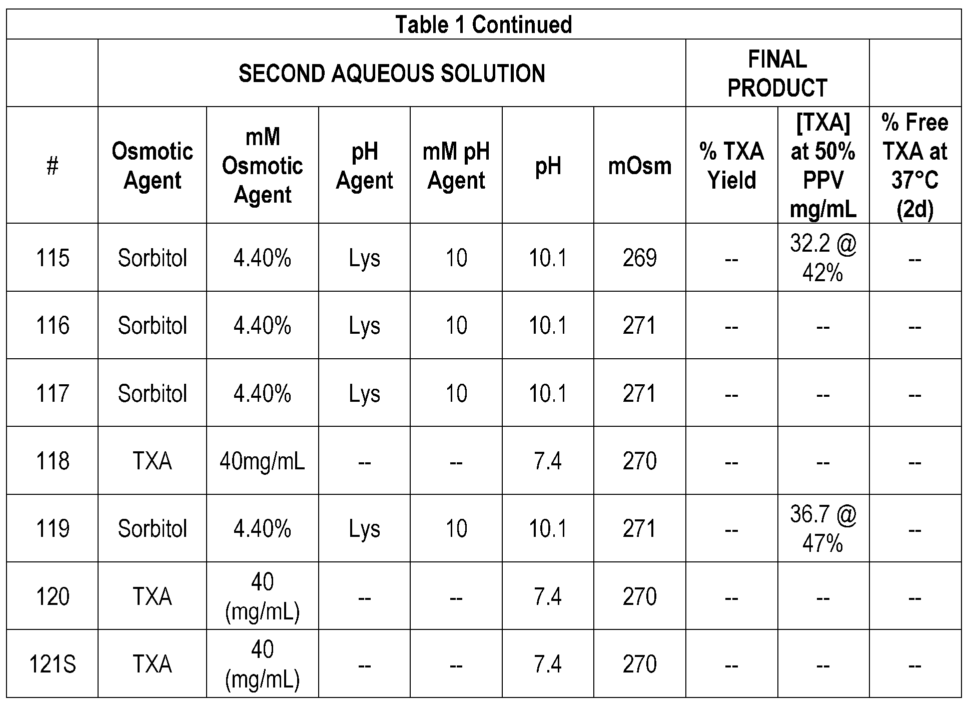

CFM is Chloroform (CFM).

DCM is Dichloromethane (CH₂Cl₂).

Lys is Lysine.

His is Histidine.

Osm/D refers to Osmotic/Density Modifying Agent.

EXP is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (26.72 mM, 10.34 mg/mL); TC (tricaprylin, 9 mM, 4.32 mg/mL); and water (0.07%).

EXP-60 is comprised of DEPC (1,2-dierucoyl-*sn*-glycero-3-phosphocholine, 12 mM, 10.67 mg/mL); DPPG (1,2-dipalmitoyl-*sn*-glycero-3-phospho-rac-(1-glycerol), 2.12 mM, 1.58 mg/mL); cholesterol (16.03 mM, 6.20 mg/mL); TC (tricaprylin, 5.40 mM, 2.59 mg/mL); and water (0.04%).

EXP-150 is comprised of DEPC (1,2-dierucoyl-*sn*-glycero-3-phosphocholine, 30 mM, 26.67 mg/mL); DPPG (1,2-dipalmitoyl-*sn*-glycero-3-phospho-rac-(1-glycerol), 5.31 mM, 3.96 mg/mL); cholesterol (40.08 mM, 15.51 mg/mL); TC (tricaprylin, 13.50 mM, 6.48 mg/mL); and water (0.11%).

EXP-C150 is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (40.08 mM, 15.51 mg/mL); TC (tricaprylin, 9 mM, 4.32 mg/mL); and water (0.07%).

EXP-C75 is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (20.04 mM, 7.76 mg/mL); TC (tricaprylin, 9 mM, 4.32 mg/mL); and water (0.07%).

EXP-C113 is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (30.19 mM, 11.68 mg/mL); TC (tricaprylin, 9 mM, 4.32 mg/mL); and water (0.07%).

EXP-DEPC50 is comprised of DEPC (1,2-dierucoyl-*sn*-glycero-3-phosphocholine, 10 mM, 8.89 mg/mL); DPPG (1,2-dipalmitoyl-*sn*-glycero-3-phospho-rac-(1-glycerol), 3.54 mM, 2.64 mg/mL); cholesterol (26.72 mM, 10.34 mg/mL); TC (tricaprylin, 9 mM, 4.32 mg/mL); and water (0.07%).

EXP-DEPC150 is comprised of DEPC (1,2-dierucoyl-*sn*-glycero-3-phosphocholine, 30 mM, 26.67 mg/mL); DPPG (1,2-dipalmitoyl-*sn*-glycero-3-phospho-rac-(1-glycerol), 3.54 mM, 2.64 mg/mL); cholesterol (26.72 mM, 10.34 mg/mL); TC (tricaprylin, 9 mM, 4.32 mg/mL); and water (0.07%).

EXP-TC150 is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (26.72 mM, 10.34 mg/mL); TC (tricaprylin, 13.5 mM, 6.48 mg/mL); and water (0.07%).

EXP-TC50 is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (26.72 mM, 10.34 mg/mL); TC (tricaprylin, 4.5 mM, 2.16 mg/mL); and water (0.07%).

EXP-DOPC100 is comprised of DOPC (dioleoyl phosphatidylcholine, 20 mM); DPPG (1,2-dipalmitoyl-*sn*-glycero-3-phospho-rac-(1-glycerol), 3.54 mM, 2.64 mg/mL); cholesterol (26.72 mM, 10.34 mg/mL); TC (tricaprylin, 9 mM, 4.32 mg/mL); and water (0.07%).

EXP-TO100 is comprised of DEPC (1,2-dierucoyl-*sn*-glycero-3-phosphocholine, 20 mM, 17.78 mg/mL); DPPG (1,2-dipalmitoyl-*sn*-glycero-3-phospho-rac-(1-glycerol), 3.54 mM, 2.64 mg/mL); cholesterol (26.72 mM, 10.34 mg/mL); TO (triolein, 9 mM); and water (0.07%).

OBLT is comprised of DEPC (1,2-dierucoyl-*sn*-glycero-3-phosphocholine, 26 mM, 23.71 mg/mL); DPPG (1,2-dipalmitoyl-*sn*-glycero-3-phospho-rac-(1-glycerol), 11 mM, 8.34 mg/mL); cholesterol (40 mM, 15.48 mg/mL); TC (tricaprylin, 40 mM, 18.84 mg/mL); and water (0.39%).

OBLT-DPPG50 is comprised of DEPC (1,2-dierucoyl-*sn*-glycero-3-phosphocholine, 26 mM, 23.71 mg/mL); DPPG (1,2-dipalmitoyl-*sn*-glycero-3-phospho-rac-(1-glycerol), 5.50 mM, 4.17 mg/mL); cholesterol (40 mM, 15.48 mg/mL); TC (tricaprylin, 40 mM, 18.84 mg/mL); and water (0.39%).

OBLT-DEPC50 is comprised of DEPC (1,2-dierucoyl-*sn*-glycero-3-phosphocholine, 13 mM, 11.86 mg/mL); DPPG (1,2-dipalmitoyl-*sn*-glycero-3-phospho-rac-(1-glycerol), 11 mM, 8.34 mg/mL); cholesterol (40 mM, 15.48 mg/mL); TC (tricaprylin, 40 mM, 18.84 mg/mL); and water (0.39%).

OBLT-DEPC150 is comprised of DEPC (1,2-dierucoyl-*sn*-glycero-3-phosphocholine, 39 mM, 35.57 mg/mL); DPPG (1,2-dipalmitoyl-*sn*-glycero-3-phospho-rac-(1-glycerol), 11 mM, 8.34 mg/mL); cholesterol (40 mM, 15.48 mg/mL); TC (tricaprylin, 40 mM, 18.84 mg/mL); and water (0.39%).

OBLT-TC50 is comprised of DEPC (1,2-dierucoyl-*sn*-glycero-3-phosphocholine, 26 mM, 23.71 mg/mL); DPPG (1,2-dipalmitoyl-*sn*-glycero-3-phospho-rac-(1-glycerol), 11 mM, 8.34 mg/mL); cholesterol (40 mM, 15.48 mg/mL); TC (tricaprylin, 20 mM, 9.42 mg/mL); and water (0.39%).

OBLT-TC150 is comprised of DEPC (1,2-dierucoyl-*sn*-glycero-3-phosphocholine, 26 mM, 23.71 mg/mL); DPPG (1,2-dipalmitoyl-*sn*-glycero-3-phospho-rac-(1-glycerol), 11 mM, 8.34 mg/mL); cholesterol (40 mM, 15.48 mg/mL); TC (tricaprylin, 60 mM, 28.26 mg/mL); and water (0.39%).

OBLT-C75 is comprised of DEPC (1,2-dierucoyl-*sn*-glycero-3-phosphocholine, 26 mM, 23.71 mg/mL); DPPG (1,2-dipalmitoyl-*sn*-glycero-3-phospho-rac-(1-glycerol), 11 mM, 8.34 mg/mL); cholesterol (30 mM, 11.61 mg/mL); TC (tricaprylin, 40 mM, 18.84 mg/mL); and water (0.39%).

Three TXA formulations are prepared as described in the Table 2 below. The encapsulated TXA in multivesicular liposomes was prepared following the similar procedure as described in Formulation #34. In addition, DEPO-TXA formulation was prepared by adding an aqueous solution of free TXA to the TXA-MVL particles to achieve 63% free TXA in the final composition.

| Table 2. | | | |
|---|---|---|---|
| Description | TXA-MVL | DEPOTXA | TXA in saline |
| [TXA], mg/mL: | 15 | 41.5 | 20 |
| Dose vol (mL): | 0.5 | 0.5 | 0.5 |
| Dose (mg): | 7.5 | 20.75 | 7.5 |
| Percent free TXA: | 5.4 | 62.5 | 100 |

Pharmacokinetic studies of the subcutaneous dosing of the above-mentioned TXA formulations were conducted in male Sprague-Dawley rats (300 to 325 grams) supplied by Charles River Labs. A total of 24 rats were used in the study, divided into 6 groups (N = 4 rats per group). Pre-dose sample collection and shaving of the legs may be performed prior to the day of dosing. The rats were administered by subcutaneous injection into the medial portion of the left hind limb, closer to the back of the rat, with a 1cc disposable syringe equipped with a 25 gauge hypodermic needle. The injection volume is 0.5 mL.

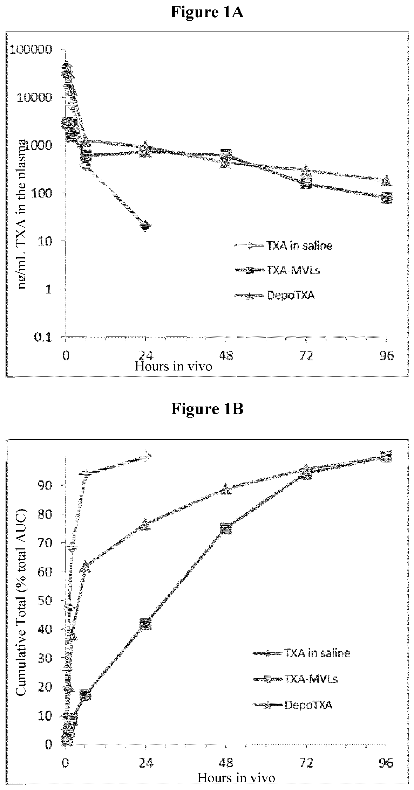

Plasma sample were collected at different times points (pre-dose, 0.5, 1, 2, 6, 24, 48, 72 & 96 hour post dose) for analysis. Blood samples were collected via the right saphenous vein using a 19 gauge needle prick or cardiac puncture for the final time point, placed into chilled tubes containing the appropriate anticoagulant, inverted several times to mix, protected from light, and kept on ice until centrifugation. The decrease in plasma levels of free TXA and the percent of total area under the curve (AUC) of free TXA were illustrated in Figs. 1A and 1B.

### Example 2: Spray Process Synthesis of DEPO-TXA

Example 2 describes a large scale synthesis of DEPO-TXA formulation (121S) where the lipid solution comprises 11.85 mg/mL DEPC, 8.34 mg/mL DPPG, 15.48 mg/mL cholesterol, and 18.84 mg/mL tricaprylin.

The lipid solution was prepared by dissolving 77.4 g cholesterol, 41.7 g DPPG, 94.2 g tricaprylin, 59.3 g DEPC and 19.5 g water in a chloroform solution. Mix the solution until solution is clear and the lipids remain in solution. To prepare the first aqueous solution, 200.0 g tranexamic acid, 300.0 g Dextran, and 4000.0 g water were mixed together. The pH of the mixture was measured to be around 7.30-7.50. Then 85% H₃PO₄ was used to titrate the mixture until pH of the first aqueous solution was approximately 5.50. The lipid solution was mixed with the first aqueous solution to form a water-in-oil emulsion. The second aqueous solution was prepared by mixing 400.0 g tranexamic acid in 10000.0 g water in a tared 20 L container. The water-in-oil emulsion was subsequently dispersed into the second aqueous solution. A rinse solution containing 400.0g tranexamic acid was prepared similarly as the second aqueous solution. After removing the chloroform, the TXA encapsulated MVLs were isolated and washed with the rinse solution, resulting in the DEPO-TXA formulation.

### Example 3: Evaluation of Toxicity of Subcutaneous Administration

The objectives of this study were to determine the potential toxicity of TXA formulations, when given by subcutaneous injection to the beagle dog and to evaluate the potential reversibility of any findings, in comparison to the Reference Control Item, Tranexamic acid. In addition, the toxicokinetic profiles were determined.

The study design was described as follows:

The Reference Item, 0.9% sodium chloride for injection, USP, was dispensed on dosing days for administration to Group 1 control animals.

The Reference Control Item (tranexamic acid) dosing formulation was prepared prior to dosing at appropriate concentrations to meet dose level requirements, using Sterile Water for Injection, USP. The formulation was filtered using 0.22 µm size filter prior to usage. On the first dose formulation occasion, the pH was determined (and was at 7.41) and one sample (6 mL) was collected in an appropriate sized container (glass or polypropylene), kept under ambient conditions, for determination of osmolality (and was at 245 mOsm/kg). The dosing formulations were dispensed for dosing of Group 2 animals.

The DEPO-TXA formulations (Test Items) were prepared as described herein (*see, e.g.,* Table 1, Formulation No. 34 and paragraph [0020]) with additional free TXA added to the TXA-MVL compositions. Two different DEPO-TXA formulations (supernatant concentration at 40 mg/mL and 120 mg/mL) were prepared, as exemplified in Table 3 below. For the 40 mg/mL formulation, % PPV is about 50.0% and the assumed interstitial volume (mL) is about 20%, rendering about 62% free TXA outside of the multivesicular liposomes. One objective of the DEPO-TXA formulation is to reduce bleeding both immediately following surgery, and over the next several days after surgery. The free fraction appears immediately in the plasma, and the encapsulated fraction appears more slowly over a three-day period. After injection of DEPO-TXA, the free fraction of the TXA appears immediately in the plasma and is cleared exactly as it is in the bolus TXA group. Also immediately after injection, the encapsulated TXA fraction begins to release slowly from the MVLs. During the first 12h, the released TXA contributes to the initial TXA peak measured in the plasma. Between 12-24h, as the free TXA is cleared quickly, the contribution of the TXA released from the MVLs into the plasma becomes apparent. The encapsulated TXA continues to be released at a rate of 5-10% per time interval over the next several days. The formulations were removed from the refrigerator and allowed to warm to room temperature for at least 30 minutes before dosing to Group 3 and Group 4 animals. The bottle was grasped in the hand gently and inverted 20-30 times via rotation of the wrist until the appearance of a uniform suspension was attained. The Test Item was NOT shaken, vortexed or stirred.

Samples of dose formulation from Reference Control Item were collected for analysis on Day 7 (from preparation for female dosing only) and on Day 10 preparations. Duplicate sets of samples (5 mL) were taken from the preparation vessel and following filtration. All samples to be analyzed were kept in a refrigerator set to maintain 4°C, out of direct light, prior to analysis.

Stability of the TXA formulation was analyzed in parallel with the study, and time points appropriate for the duration of the study was analyzed at completion of the in-life phase.

21 male and 21 female beagle dogs were used in this study. The animals were from 6 to 7 months old and weighed between 7.5 and 9.8 kg (males) and 6.0 and 8.2 kg (females) at initiation of dosing.

**Table 3: Experimental Design**

| **Group No.** | **Test Material** | **Dose Level (mg/kg/dose)** | **Dose Volume (mL/kg)** | **Dose Concentration (mg/mL)** | **No. of Animals** | | | |
|---|---|---|---|---|---|---|---|---|
| | | | | | **Main Study** | | **Recovery Study** | |
| | | | | | **Males** | **Females** | **Males** | **Females** |
| 1 | Reference Item | 0 | 3 | 0 | 3 | 3 | 2 | 2 |
| 2 | TXA | 120 | 3 | 40 | 3 | 3 | 2 | 2 |
| 3 | DEPO-TXA | 40 | 1.03 | 38.8 | 3 | 3 | 2 | 2 |
| 4 | DEPO-TXA | 120 | 3.09 | 38.8 | 3 | 3 | 2 | 2 |

The Test, Reference Control and Reference Items were administered to the appropriate animals via subcutaneous injection into the scapular and mid-dorsal areas on Days 1, 4, 7, and 10. The dose volume for each animal was based on the most recent body weight measurement. The volume for each dose was administered using a syringe/needle over one injection. Injection sites were rotated between two delimited sites (and the same site for all animals on a given day). Dosing area was shaved as needed and injection sites were marked with a pen (target area of 5 cm x 5 cm). The first dosing site was in the mid-scapular region of the back and the second dosing site was caudal to the first site in the mid-dorsum region.

Blood was collected from the jugular vein. Urine was collected overnight from individually housed animals. After collection, samples were transferred to the appropriate laboratory for processing. Animals were fasted overnight before blood sampling (for clinical chemistry). Animals were deprived of food and water during the urine collection procedure.

Blood was collected from the jugular vein (or cephalic vein) from all animals in lithium heparin-containing tubes. Samples were collected serially, on Days 1 and 10, at the following time points: pre-dose, 15 min, 30 min, 1, 2, 6, 12, 24, 36, 48, and 72 hours post-dose.

Samples were mixed gently and placed on crushed wet ice until centrifugation, which was carried out as soon as practical. The samples were centrifuged for 10 minutes in a refrigerated centrifuge (set to maintain the temperature at 4°C) at 2700 rpm. The resultant plasma was separated, transferred to uniquely labeled clear polypropylene tubes, and frozen immediately over dry ice and transferred to a freezer set to maintain -80°C.

Plasma samples were analyzed for concentration of the tranexamic acid using a validated analytical procedure.

Toxicokinetic parameters were generated from the test item (tranexamic acid) individual concentrations in plasma from Days 1 and 10, whenever practical.

### Results and Discussion

Subcutaneous injection of 40 and 120 mg/kg/dose of DEPO-TXA ("Test Item") to Beagle dogs over a period of 14 days was well-tolerated and did not result in any adverse toxicity.

Dose-related minor clinical signs, such as slight and occasional emesis, were observed in animals dosed with DEPO-TXA following dosing. These clinical signs were also observed in animals dosed with TXA, at a similar incidence and severity. No Test Item or Reference Control Item-related clinical signs were noted on days between dosing, nor during the recovery period.

There were no effects on body weight, body weight gain, food intake, ophthalmology, electrocardiology, hematology, coagulation, D-dimer content, urinalysis parameters, organ weights or macroscopic findings compared to control groups.

Toxicokinetic analysis clearly demonstrated that high levels of the TXA contained in the DEPO-TXA dosing material were absorbed into peripheral circulation following subcutaneous injection. There were no differences between the genders. Plasma exposures (Cₘₐₓ and AUC) were equivalent on treatment days 1 and 10.

**Table 4. Summary of Toxicokinetic Parameters**

| **Dose (mg/kg/dose)** | **Day** | **Tₘₐₓ (hr)** | **Cₘₐₓ (µg/mL)** | **AUC₀₋₇₂ (hr•µg/mL)** | **t_{1/2} (hr)** |
|---|---|---|---|---|---|
| 120 mg/kg/dose TXA | 1 | 1.2 | 140 | 490 | 25.7 |
| | 10 | 1.1 | 119 | 484 | 28.9 |
| 40 mg/kg/dose DEPO-TXA | 1 | 0.63 | 48.9 | 188 | 28.5 |
| | 10 | 0.55 | 50.8 | 182 | 22.9 |
| 120 mg/kg/dose DEPO-TXA | 1 | 1.0 | 109 | 526 | 27.9 |
| | 10 | 0.68 | 138 | 524 | 21.7 |

On Day 1 following DepoTXA administration, mean ± standard deviation (n=10) genders combined plasma concentrations of TXA peaked at 0.63±0.27 and 1.0±0.0 hours (Tmax) after dosing for the 40 and 120 mg/kg dose groups, respectively. Respective mean peak levels (Cmax) were 48.9±10.8 and 109±12.7 µg/mL. On Day 10, respective mean peak concentrations were reached at 0.55±0.16 and 0.68±0.29 hours, with mean peak levels at 50.8±9.8 and 138±15.1 µg/mL for low and high dose animals, representing minimal change in peak exposure after dosing on Days 1, 4, 7 and 10. Values for genders combined mean ± st. dev. (n=10) for AUC0-24, AUC0-48, AUC0-72 and AUC0-inf at the 40 mg/kg DepoTXA dose level on Day 1 were 159±15.7, 177±18.0, 188±18.5 and 201±17.2 µg·hr/mL, respectively, and for the 120 mg/kg DepoTXA dose level were 445±35.9, 498±35.3, 526±34.6 and 561±35.9 µg·hr/mL. On Day 10, respective mean values for AUC0-24, AUC0-48 and AUC0-72 at the 40 mg/kg dose level were 156±15.7, 173±17.5, 182±18.3 µg·hr/mL, and at the 120 mg/kg dose level were 455±45.0, 503±50.6 and 524±52.8 µg·hr/mL. Mean terminal elimination half-life (t1/2elim) for DepoTXA on Day 1 for the 40 and 120 mg/kg dose levels was 28.5±7.4 and 27.9±9.8 hours, respectively, and on Day 10 were 22.9±6.8 and 21.7±5.7.

For free TXA (Reference Control Item given to Group 2 animals), on Day 1, mean ± standard deviation (n=10) genders combined plasma concentrations of TXA peaked at 1.2±0.42 hours (Tmax) after dosing at 120 mg/kg, with mean peak levels (Cmax) at 140±19.2 µg/mL. On Day 10, mean peak concentrations were reached at 1.1±0.32 hours, with mean peak levels at 119±15.4 µg/mL. Values for genders combined mean ± st. dev. (n=10) for AUC0-24, AUC0-48, AUC0-72 and AUC0-inf on Day 1 following TXA were 475±35.1, 485±36.1, 490±36.5 and 495±36.8 µg·hr/mL, respectively. On Day 10, respective mean values for AUC0-24, AUC0-48 and AUC0-72 were 468±42.5, 479±44.2 and 484±45.2 µg·hr/mL. Mean terminal elimination half-life (t1/2elim) for TXA on Day 1 was 25.7±2.0 hours and on Day 10 was 28.9±2.9.

Comparing the 120 mg/kg dose level of DepoTXA to free TXA, mean peak exposures (Cmax) were 28% higher for the free TXA group on Day 1, but peak TXA levels were 14% lower on study Day 10 following the fourth and final subcutaneous dose. Mean overall systemic exposures (AUC0-72) on Day 1 were 7% greater for the DepoTXA group vs. the free TXA group, and on Day 10 mean exposures were 8% greater for the DepoTXA group.

DepoTXA was administered subcutaneously to dogs at 40 and 120 mg/kg/dose on study Days 1, 4, 7 and 10. Following the first and last dose blood samples were collected out to 72 hours after dosing and toxicokinetic parameter estimates were determined. Results of the TK analysis clearly demonstrated that high levels of the TXA contained in the DepoTXA dosing material were absorbed into peripheral circulation following subcutaneous injection. Administration of the Depo-TXA was well-tolerated, with no adverse toxicity. Plasma exposures (Cmax and AUC) were equivalent on Days 1 and 10. Comparing mean exposures between the 120 mg/kg DepoTXA group and the 120 mg/kg free TXA group, mean peak (Cmax) exposures were higher for the free TXA group after a single dose (Day 1), but after multiple doses (Day 10) peak mean exposures were higher for the DepoTXA group. Mean overall systemic exposures (AUC0-72) were slightly greater for the DepoTXA group.

Fig. 2 illustrates mean plasma concentrations of TXA over 72 hours after administration on treatment day 1. Group 2 was administered the Reference Control Item, Group 3 was administered 40 mg/kg Depo-TXA, and Group 4 was administered 120 mg/kg Depo-TXA.

Fig. 3 illustrates mean plasma concentrations of TXA over 72 hours after administration on treatment day 10. Group 2 was administered the Reference Control Item, Group 3 was administered 40 mg/kg Depo-TXA, and Group 4 was administered 120 mg/kg Depo-TXA.

Fig. 4 illustrates the concentration of TXA in plasma over 12 hours after administration on treatment day 1.

Fig. 5A illustrates the percent of total area under the curve (AUC) of TXA for up to 72 hours post-injection on treatments day 1. Fig. 5B illustrates the percent of total area under the curve (AUC) of TXA for up to 72 hours post-injection on treatments day 10. Specifically, in the free TXA group, 94% of the administered TXA is cleared after 12 hours, whereas in the DepoTXA group, 25% of the dose remains at 12 hours, and is delivered over the next 60 hours.

Fig. 6A illustrates the total amount of TXA delivered over 72 hours after administration on treatment day 1. Fig. 6B illustrates the total amount of TXA delivered over 72 hours after administration on treatment day 10. Administration of TXA alone results in exposure to nearly the full TXA dose in less than 24 hours. In contrast, Depo-TXA provides, for example, exposure of 100 mg/kg TXA (from a 120 mg/kg dose) at 24 hours, demonstrating the sustained release profile of Depo-TXA.

Fig. 7A illustrates the decrease in plasma levels of TXA over 72 hours after administration on day 1. Fig. 7B illustrates the decrease in plasma levels of TXA over 72 hours after administration on day 10.

Thus, after injection of DepoTXA, the free fraction of the TXA appears immediately in the plasma and is cleared exactly as it is in the bolus TXA group. Also immediately after injection, the encapsulated TXA fraction begins to release slowly. During the first 12h, the released TXA contributes to the initial TXA peak measured in the plasma. Between 12- 24h, as the free TXA is cleared quickly, the contribution of the released TXA to the measured plasma TXA levels becomes apparent. The encapsulated TXA continues to be released at a rate of 5-10% per time interval over the next 24-72 hours, as shown in Figures 8A and 8B.

In absence of adverse effects at both doses of DEPO-TXA, the no observed adverse effect level (NOAEL) in this study was considered to be 120 mg/kg/dose DEPO-TXA (with a gender combined mean plasma AUC₀₋₇₂ of 524 µg•h/mL and a Cₘₐₓ of 138 µg/mL on Day 10).

## Claims

1. A pharmaceutical composition comprising:
multivesicular liposomes encapsulating tranexamic acid, said multivesicular liposomes comprising: tranexamic acid; a lipid component comprising at least one amphipathic lipid and at least one neutral lipid; and one or more pH modifying agents.

2. The pharmaceutical composition of Claim 1, further comprising unencapsulated tranexamic acid.

3. The pharmaceutical composition of Claim 1 or 2, wherein the multivesicular liposomes further comprise one or more osmotic agents and/or density modifying agents.

4. The pharmaceutical composition of any one of Claims 1 to 3, wherein the multivesicular liposomes further comprise cholesterol and/or a plant sterol.

5. The pharmaceutical composition of any one of Claims 1 to 4, wherein the amphipathic lipid comprises phosphatidylcholine, or phosphatidylglycerol or salts thereof, or combinations thereof.

6. The pharmaceutical composition of Claim 5, wherein the phosphatidylglycerol is DPPG.

7. The pharmaceutical composition of Claim 5, wherein the phosphatidylcholine is selected from DEPC or DOPC, or a combination thereof.

8. The pharmaceutical composition of any one of Claims 1 to 7, wherein the neutral lipid comprises triglyceride, propylene glycol ester, ethylene glycol ester, or squalene, or combinations thereof.

9. The pharmaceutical composition of Claim 8, wherein the neutral lipid comprises triglyceride.

10. The pharmaceutical composition of Claim 8 or 9, wherein the triglyceride is selected from triolein or tricaprylin, or a combination thereof.

11. The pharmaceutical composition of any one of Claims 1 to 10, wherein said pH modifying agents are selected from organic acids, organic bases, inorganic acids, or inorganic bases, or combinations thereof.

12. The pharmaceutical composition of Claim 11, wherein the inorganic acid is selected from hydrochloric acid or phosphoric acid.

13. The pharmaceutical composition of Claim 11, wherein the organic acid is selected from tartaric acid, or glutamic acid, or a combination thereof.

14. The pharmaceutical composition of any one of Claims 11 to 13, wherein the organic base is selected from histidine, arginine, lysine, or tromethamine, or combinations thereof.

15. The pharmaceutical composition of any one of Claims 1 to 14, wherein the concentration of total tranexamic acid in the pharmaceutical composition is from about 1 mg/mL to about 80 mg/mL.

16. The pharmaceutical composition of any one of Claims 1 to 15, wherein the concentration of total tranexamic acid in the pharmaceutical composition is from about 5 mg/mL to about 25 mg/mL.

17. The pharmaceutical composition of any one of Claims 2 to 16, wherein the unencapsulated tranexamic acid is about 1% to about 80% of the total amount of tranexamic acid in the pharmaceutical composition.

18. The pharmaceutical composition of Claim 17, wherein the unencapsulated tranexamic acid is about 50% of the total amount of tranexamic acid in the pharmaceutical composition.

19. The pharmaceutical composition of any one of Claims 2 to 16, wherein the unencapsulated tranexamic acid is less than about 10% of the total tranexamic acid in the pharmaceutical composition.

20. The pharmaceutical composition of any one of Claims 1 to 19, wherein said multivesicular liposomes have an external pH range from about 4.0 to about 9.0.

21. The pharmaceutical composition of any one of 1 to 20, wherein said multivesicular liposomes have an internal pH range of about 3.0 to about 9.0.

22. A pharmaceutical composition of any one of Claims 1 to 21 for use in treating, ameliorating or preventing blood loss.

23. The pharmaceutical composition for use according to Claim 22, wherein the pharmaceutical composition is for parenteral, topical or both topical and parenteral administration.

24. The pharmaceutical composition for use according to Claim 23, wherein the parenteral administration is selected from subcutaneous injection, tissue injection, wound infiltration, or wound instillation.

25. The pharmaceutical composition for use according to Claim 23, wherein the topical administration is through direct contact of said pharmaceutical composition with a cavity or a surface of the subject body that is in need of treatment.

26. A process for preparing multivesicular liposomes comprising tranexamic acid, said process comprising:
preparing a first aqueous component comprising tranexamic acid and at least one pH modifying agent;
preparing a lipid component comprising at least one organic solvent, at least one amphipathic lipid, and at least one neutral lipid;
mixing said first aqueous component and said lipid component to form a water-in-oil emulsion, wherein at least one component comprises tranexamic acid;
contacting said water-in-oil emulsion with a second aqueous component to form solvent-containing spherules; and
removing the organic solvent from the solvent-containing spherules to form multivesicular liposomes.

27. The process of Claim 26, further comprising suspending the multivesicular liposomes in a solution comprising tranexamic acid to form a pharmaceutical composition comprising both encapsulated and unencapsulated tranexamic acid.

28. The process of Claim 26, further comprising suspending the multivesicular liposomes in a solution comprising saline to form a pharmaceutical composition comprising encapsulated tranexamic acid.

## Patentansprüche

1. Pharmazeutische Zusammensetzung, umfassend:
multivesikuläre Liposomen, die Tranexamsäure einkapseln, wobei die multivesikulären Liposomen umfassen: Tranexamsäure; eine Lipidkomponente, die mindestens ein amphipathisches Lipid und mindestens ein neutrales Lipid umfasst; und ein oder mehrere pHmodifizierende Mittel.

2. Pharmazeutische Zusammensetzung nach Anspruch 1, die zudem nicht eingekapselte Tranexamsäure umfasst.

3. Pharmazeutische Zusammensetzung 1 oder 2, wobei die multivesikulären Liposomen zudem ein oder mehrere osmotische Mittel und/oder dichtemodifizierende Mittel umfassen.

4. Pharmazeutische Zusammensetzung nach einem der Ansprüche 1 bis 3, wobei die multivesikulären Liposomen zudem Cholesterin und/oder ein Pflanzensterin umfassen.

5. Pharmazeutische Zusammensetzung nach einem der Ansprüche 1 bis 4, wobei das amphipatische Lipid Phosphatidylcholin oder Phosphatidylglycerin oder Salze davon oder Kombinationen davon umfasst.

6. Pharmazeutische Zusammensetzung nach Anspruch 5, wobei das Phosphatidylglycerin DPPG ist.

7. Pharmazeutische Zusammensetzung nach Anspruch 5, wobei das Phosphatidylcholin aus DEPC oder DOPC oder einer Kombination davon ausgewählt ist.

8. Pharmazeutische Zusammensetzung nach einem der Ansprüche 1 bis 7, wobei das neutrale Lipid Triglycerid, Propylenglycolester, Ethylenglycolester oder Squalen oder Kombinationen davon umfasst.

9. Pharmazeutische Zusammensetzung nach Anspruch 8, wobei das neutrale Lipid Triglycerid umfasst.

10. Pharmazeutische Zusammensetzung nach Anspruch 8 oder 9, wobei das Triglycerid aus Triolein oder Tricaprylin oder einer Kombination davon ausgewählt ist.

11. Pharmazeutische Zusammensetzung nach einem der Ansprüche 1 bis 10, wobei die pH-modifizierenden Mittel aus organischen Säuren, organischen Basen, anorganischen Säuren oder anorganischen Basen oder Kombinationen davon ausgewählt sind.

12. Pharmazeutische Zusammensetzung nach Anspruch 11, wobei die anorganische Säure aus Salzsäure oder Phosphorsäure ausgewählt ist.

13. Pharmazeutische Zusammensetzung nach Anspruch 11, wobei die organische Säure aus Weinsäure oder Glutaminsäure oder einer Kombination davon ausgewählt ist.

14. Pharmazeutische Zusammensetzung nach einem der Ansprüche 11 bis 13, wobei die organische Base aus Histidin, Arginin, Lysin oder Tromethamin oder Kombinationen davon ausgewählt ist.

15. Pharmazeutische Zusammensetzung nach einem der Ansprüche 1 bis 14, wobei die Konzentration der gesamten Tranexamsäure in der pharmazeutischen Zusammensetzung von etwa 1 mg/ml bis etwa 80 mg/ml reicht.

16. Pharmazeutische Zusammensetzung nach einem der Ansprüche 1 bis 15, wobei die Konzentration der gesamten Tranexamsäure in der pharmazeutischen Zusammensetzung von etwa 5 mg/ml bis etwa 25 mg/ml reicht.

17. Pharmazeutische Zusammensetzung nach einem der Ansprüche 2 bis 16, wobei die nicht eingekapselte Tranexamsäure etwa 1% bis etwa 80% der Gesamtmenge an Tranexamsäure in der pharmazeutischen Zusammensetzung ausmacht.

18. Pharmazeutische Zusammensetzung nach Anspruch 17, wobei die nicht eingekapselte Tranexamsäure etwa 50% der Gesamtmenge an Tranexamsäure in der pharmazeutischen Zusammensetzung ausmacht.

19. Pharmazeutische Zusammensetzung nach einem der Ansprüche 2 bis 16, wobei die nicht eingekapselte Tranexamsäure weniger als etwa 10% der gesamten Tranexamsäure in der pharmazeutischen Zusammensetzung ausmacht.

20. Pharmazeutische Zusammensetzung nach einem der Ansprüche 1 bis 19, wobei die multivesikulären Liposomen einen äußeren pH-Bereich von etwa 4,0 bis etwa 9,0 aufweisen.

21. Pharmazeutische Zusammensetzung nach einem der Ansprüche 1 bis 20, wobei die multivesikulären Liposomen einen inneren pH-Bereich von etwa 3,0 bis etwa 9,0 aufweisen.

22. Pharmazeutische Zusammensetzung zur Verwendung nach einem der Ansprüche 1 bis 21 zur Verwendung bei der Behandlung, Verbesserung oder Prävention von Blutverlust.

23. Pharmazeutische Zusammensetzung zur Verwendung nach Anspruch 22, wobei die pharmazeutische Zusammensetzung der parenteralen, topischen oder sowohl topischen als auch parenteralen Verabreichung dient.

24. Pharmazeutische Zusammensetzung zur Verwendung nach Anspruch 23, wobei die parenterale Verabreichung aus subkutaner Injektion, Gewebeinjektion, Wundinfiltration oder Wundinstillation ausgewählt ist.

25. Pharmazeutische Zusammensetzung zur Verwendung nach Anspruch 23, wobei die topische Verabreichung durch direkten Kontakt der pharmazeutischen Zusammensetzung mit einem Hohlraum oder einer Oberfläche des Körpers des Individuums, das eine Behandlung benötigt, erfolgt.

26. Verfahren zur Herstellung multivesikulärer Liposomen, die Tranexamsäure umfassen, wobei das Verfahren umfasst:
Herstellen einer ersten wässrigen Komponente, die Tranexamsäure und mindestens ein pH-modifizierendes Mittel umfasst;
Herstellen einer Lipidkomponente, die mindestens ein organisches Lösungsmittel, mindestens ein amphipathisches Lipid und mindestens ein neutrales Lipid umfasst;
Mischen der ersten wässrigen Komponente und der Lipidkomponente, so dass eine Wasser-in-Öl-Emulsion gebildet wird, wobei mindestens eine Komponente Tranexamsäure umfasst;
Inkontaktbringen der Wasser-in-Öl-Emulsion mit einer zweiten wässrigen Komponente, so dass lösungsmittelhaltige Kügelchen gebildet werden; und
Entfernen des organischen Lösungsmittels aus den lösungsmittelhaltigen Kügelchen, so dass multivesikuläre Liposomen gebildet werden.

27. Verfahren nach Anspruch 26, zudem umfassend das Suspendieren der multivesikulären Liposomen in einer Tranexamsäure umfassenden Lösung zur Bildung einer pharmazeutischen Zusammensetzung, die sowohl eingekapselte als auch nicht eingekapselte Tranexamsäure enthält.

28. Verfahren nach Anspruch 26, zudem umfassend das Suspendieren der multivesikulären Liposomen in einer Salzlösung umfassenden Lösung zur Bildung einer pharmazeutischen Zusammensetzung, die eingekapselte Tranexamsäure umfasst.

## Revendications

1. Composition pharmaceutique comprenant :
des liposomes multivésiculaires encapsulant de l'acide tranexamique, lesdits liposomes multivésiculaires comprenant : de l'acide tranexamique ; un composant lipidique comprenant au moins un lipide amphipathique et au moins un lipide neutre ; et un ou plusieurs agents de modification du pH.

2. Composition pharmaceutique selon la revendication 1, comprenant en outre de l'acide tranexamique non encapsulé.

3. Composition pharmaceutique selon la revendication 1 ou 2, dans laquelle les liposomes multivésiculaires comprennent en outre un ou plusieurs agents osmotiques et/ou agents de modification de masse volumique.

4. Composition pharmaceutique selon l'une quelconque des revendications 1 à 3, dans laquelle les liposomes multivésiculaires comprennent en outre du cholestérol et/ou un stérol végétal.

5. Composition pharmaceutique selon l'une quelconque des revendications 1 à 4, dans laquelle le lipide amphipathique comprend de la phosphatidylcholine, ou du phosphatidylglycérol ou des sels de ceux-ci, ou des combinaisons de ceux-ci.

6. Composition pharmaceutique selon la revendication 5, dans laquelle le phosphatidylglycérol est DPPG.

7. Composition pharmaceutique selon la revendication 5, dans laquelle la phosphatidylcholine est choisie parmi DEPC ou DOPC, ou une combinaison de ceux-ci.

8. Composition pharmaceutique selon l'une quelconque des revendications 1 à 7, dans laquelle le lipide neutre comprend un triglycéride, un ester de propylène glycol, un ester d'éthylène glycol ou du squalène, ou des combinaisons de ceux-ci.

9. Composition pharmaceutique selon la revendication 8, dans laquelle le lipide neutre comprend un triglycéride.

10. Composition pharmaceutique selon la revendication 8 ou 9, dans laquelle le triglycéride est choisi parmi la trioléine ou la tricapryline, ou une combinaison de celles-ci.

11. Composition pharmaceutique selon l'une quelconque des revendications 1 à 10, dans laquelle lesdits agents modifiant le pH sont choisis parmi des acides organiques, des bases organiques, des acides inorganiques ou des bases inorganiques, ou des combinaisons de ceux-ci.

12. Composition pharmaceutique selon la revendication 11, dans laquelle l'acide inorganique est choisi parmi l'acide chlorhydrique ou l'acide phosphorique.

13. Composition pharmaceutique selon la revendication 11, dans laquelle l'acide organique est choisi parmi l'acide tartrique, ou l'acide glutamique, ou une combinaison de ceux-ci.

14. Composition pharmaceutique selon l'une quelconque des revendications 11 à 13, dans laquelle la base organique est choisie parmi l'histidine, l'arginine, la lysine ou la trométhamine, ou des combinaisons de celles-ci.

15. Composition pharmaceutique selon l'une quelconque des revendications 1 à 14, dans laquelle la concentration d'acide tranexamique total dans la composition pharmaceutique est d'environ 1 mg/ml à environ 80 mg/ml.

16. Composition pharmaceutique selon l'une quelconque des revendications 1 à 15, dans laquelle la concentration d'acide tranexamique total dans la composition pharmaceutique est d'environ 5 mg/ml à environ 25 mg/ml.

17. Composition pharmaceutique selon l'une quelconque des revendications 2 à 16, dans laquelle l'acide tranexamique non encapsulé est d'environ 1 % à environ 80 % de la quantité totale d'acide tranexamique dans la composition pharmaceutique.

18. Composition pharmaceutique selon la revendication 17, dans laquelle l'acide tranexamique non encapsulé est environ 50 % de la quantité totale d'acide tranexamique dans la composition pharmaceutique.

19. Composition pharmaceutique selon l'une quelconque des revendications 2 à 16, dans laquelle l'acide tranexamique non encapsulé est inférieur à environ 10 % de l'acide tranexamique total dans la composition pharmaceutique.

20. Composition pharmaceutique selon l'une quelconque des revendications 1 à 19, dans laquelle lesdits liposomes multivésiculaires ont une plage de pH externe d'environ 4,0 à environ 9,0.

21. Composition pharmaceutique selon l'une quelconque des revendications 1 à 20, dans laquelle lesdits liposomes multivésiculaires ont une plage de pH interne d'environ 3,0 à environ 9,0.

22. Composition pharmaceutique selon l'une quelconque des revendications 1 à 21, pour utilisation dans le traitement, l'amélioration ou la prévention de perte de sang.

23. Composition pharmaceutique pour utilisation selon la revendication 22, la composition pharmaceutique étant pour administration parentérale, topique ou à la fois topique et parentérale.

24. Composition pharmaceutique pour utilisation selon la revendication 23, l'administration parentérale étant choisie parmi l'injection sous-cutanée, l'injection tissulaire, l'infiltration de plaie ou l'instillation de plaie.

25. Composition pharmaceutique pour utilisation selon la revendication 23, l'administration topique étant effectuée par contact direct de ladite composition pharmaceutique avec une cavité ou une surface du corps du sujet qui a besoin d'un traitement.

26. Procédé de préparation de liposomes multivésiculaires comprenant de l'acide tranexamique, ledit procédé comprenant :
la préparation d'un premier composant aqueux comprenant de l'acide tranexamique et au moins un agent de modification du pH ;
la préparation d'un composant lipidique comprenant au moins un solvant organique, au moins un lipide amphipathique, et au moins un lipide neutre ;
le mélange dudit premier composant aqueux et dudit composant lipidique pour former une émulsion eau dans huile, au moins un composant comprenant de l'acide tranexamique ;
la mise en contact de ladite émulsion eau dans huile avec un deuxième composant aqueux pour former des sphérules contenant un solvant ; et
l'élimination du solvant organique à partir des sphérules contenant un solvant pour former des liposomes multivésiculaires.

27. Procédé selon la revendication 26, comprenant en outre la mise en suspension des liposomes multivésiculaires dans une solution comprenant de l'acide tranexamique pour former une composition pharmaceutique comprenant à la fois de l'acide tranexamique encapsulé et non encapsulé.

28. Procédé selon la revendication 26, comprenant en outre la mise en suspension des liposomes multivésiculaires dans une solution comprenant une solution saline pour former une composition pharmaceutique comprenant de l'acide tranexamique encapsulé.
